# FASCICULE DE BREVET EUROPEEN

(11) **EP 2 488 877 B1**
(45) Date de publication et mention de la délivrance du brevet: **04.01.2017**
(21) Numéro de dépôt: 10798578.0
(22) Date de dépôt: 13.10.2010
(51) Int. Cl.: G01N 33/80

(54) **PROCEDE D'OBTENTION DE BIOPROTHESES MEDICALES IMPLANTABLES AYANT DES PROPRIETES DE CALCIFICATION REDUITES**
VERFHREN ZUR HERSTELLUNG VON IMPLANTIERBAREN MEDIZINISCHEN BIOPROTHESEN MIT VERMINDERTEN VERKALKUNGSEIGENSCHAFTEN
METHOD FOR PRODUCING IMPLANTABLE MEDICAL BIOPROSTHESES HAVING REDUCED CALCIFICATION PROPERTIES

(30) Priorité: 15.10.2009 FR 0957229
(43) Date de publication de la demande: 22.08.2012
(73) Titulaire: Schussler, Olivier, 92150 Suresnes (FR)
(72) Inventeur: Schussler, Olivier, 92150 Suresnes (FR)
(74) Mandataire: Dennemeyer & Associates S.A.
(86) Numéro de dépôt international: PCT/FR2010/000683
(87) Numéro de publication internationale: WO 2011/051574

(56) Documents cités:
- BALCH C M ET AL: "BLOOD GROUP COMPATIBILITY AND AORTIC VALVE ALLO TRANSPLANTATION IN MAN", JOURNAL OF THORACIC AND CARDIOVASCULAR SURGERY, vol. 70, no. 2, 1975, pages 256-259, XP009133707, ISSN: 0022-5223
- JASHARI R ET AL: "Is ABO group incompatibility really the reason of accelerated failure of cryopreserved allografts in very young patients? - Echography assessment of the European Homograft Bank (EHB) cryopreserved allografts used for reconstruction of the right ventricular outflow tract*", CELL AND TISSUE BANKING, KLUWER ACADEMIC PUBLISHERS, DO LNKD- DOI:10.1007/S10561-004-1442-Z, vol. 5, no. 4, 1 décembre 2004 (2004-12-01), pages 253-259, XP019234032, ISSN: 1573-6814
- CHRISTENSON JAN T ET AL: "Blood group incompatibility and accelerated homograft fibrocalcifications.", THE JOURNAL OF THORACIC AND CARDIOVASCULAR SURGERY JAN 2004 LNKD- PUBMED:14752436, vol. 127, no. 1, janvier 2004 (2004-01), pages 242-250, XP002582904, ISSN: 0022-5223
- YANKAH ET AL: "Accelerated degeneration of allografts in the first two years of life", THE ANNALS OF THORACIC SURGERY, ELSEVIER, vol. 60, 1 août 1995 (1995-08-01), pages S71-S77, XP005252360, ISSN: 0003-4975
- SHADDY ROBERT E ET AL: "Immunology and failure of valved allografts in children.", THE ANNALS OF THORACIC SURGERY OCT 2002 LNKD- PUBMED:12400797, vol. 74, no. 4, octobre 2002 (2002-10), pages 1271-1275, XP002582905, ISSN: 0003-4975
- KNOSALLA C ET AL: "Surgical treatment of active infective aortic valve endocarditis with associated periannular abscess: 11 Year results", EUROPEAN HEART JOURNAL, vol. 21, no. 6, mars 2000 (2000-03), pages 490-497, XP002582906, ISSN: 0195-668X
- FEINGOLD B ET AL: "Expression of A and B Blood Group Antigens on Cryopreserved Homografts", THE ANNALS OF THORACIC SURGERY, ELSEVIER LNKD- DOI:10.1016/J.ATHORACSUR.2008.09.073, vol. 87, no. 1, 1 janvier 2009 (2009-01-01), pages 211-214, XP025838581, ISSN: 0003-4975 [extrait le 2008-12-24]

## Description

### DOMAINE DE L'INVENTION

La présente invention se rapporte au domaine dés bioprothèses médicales implantables.

### ART ANTERIEUR

Les bioprothèses implantables sont connues depuis de nombreuses années. Elles consistent principalement en des prothèses à base de tissu animal, qui sont destinées à être implantées dans le corps d'un patient afin de palier le dysfonctionnement d'un tissu, en général d'un conduit artériel ou vasculaire ou bien encore d'une valvule cardiaque.

Parmi les bioprothèses cardiaques, on connaît notamment les valves mitrales, les valves aortiques, les valves pulmonaires, les valves tricuspides ou encore les implants de réfection pariétale. Parmi les bioprothèses vasculaires, on connaît notamment les conduits aortiques ou encore les conduits pulmonaires. On connaît,aussi diverses autres bioprothèses, tels que les ligaments « artificiels » de genou, de cheville ou d'épaule, etc.

Les bioprothèses implantables sont réalisées à partir de tissus animaux, principalement de tissu d'origine bovine, porcine, ovine, ou encore les tissus provenant de kangourous, de phoques, de chameaux ou d'équidés. On utilise couramment des tissus animaux qui ont été fixés chimiquement, qui englobent les valves cardiaques elles-mêmes, des vaisseaux, la peau, la dure-mère, le péricarde, des ligaments, des tendons, la sous muqueuse digestive etc.

Les bioprothèses implantables sont utilisées avec succès chez l'homme depuis au moins quarante ans. En particulier, les bioprothèses présentent de nombreux avantages, en comparaison des prothèses mécaniques. Notamment, avec les bioprothèses, on ne rencontre pas les risques de thrombose observés avec les prothèses mécaniques. Ainsi, l'utilisation de bioprothèses offre aux patients un grand confort de vie et ne nécessitent pas de traitement à l'aide d'agents anti-coagulants susceptible de provoquer des hémorragies, contrairement aux prothèses mécaniques. De plus, avec la durée d'implantation, l'efficacité de fonctionnement des bioprothèses peut décroître progressivement, mais sans rupture brutale comme pour les prothèses mécaniques, ce qui limite considérablement les conséquences cliniques d'un dysfonctionnement pour le patient. Dé plus, des techniques chirurgicales non invasives d'implantation de bioprothèses sont aujourd'hui disponibles, en particulier pour le remplacement des valvules cardiaques.

Pour les raisons décrites ci-dessus, les bioprothèses sont actuellement les prothèses auxquelles on a le plus fréquemment recours pour pallier un dysfonctionnement, notamment cardiaque ou vasculaire.

Les bioprothèses sont prescrites le plus couramment chez les sujets jeunes, chez la femme enceinte et chez les sujets âgés.

Toutefois, le principal inconvénient des bioprothèses est leur trop faible longévité dans le corps du patient, qui dépasse rarement 15 ans. En moyenne, une bioprothèses valvulaires aortique possède une durée de vie allant de 12 à 14 ans.

La cause majeure de l'altération des bioprothèses réside dans leur dégénérescence, du fait d'une calcification progressive, qui accroît l'épaisseur du tissu bioprothétique et altère leurs propriétés mécaniques, ce qui en empêche le bon fonctionnement et peut provoquer leur rupture, notamment par déchirure (Tyers, et al. (1995) Ann Thorac Surg 60, S464-468; discussion S468-469 ; Bortolotti, et al. (1991). J Card Surg 6, 638-643; Grunkemeier, et al. (1995) J Heart Valve Dis 4, 49-55 ; Schoen, et al. (1984) Cardiol Clin 2, 717-739).

Du fait de cette durée de vie limitée, les bioprothèses sont prioritairement implantées chez les individus ayant dépassé l'âge de 50 ans. Elles sont inadaptées à une implantation chez les enfants. La bioprothèse est l'implant préféré pour les sujets âgés de plus de 70 ans (Ueyama, et al. (2002). Artif Organs 26, 1059-1062). En revanche, pour les sujets de moins de 70 ans, dont la longévité de vie est supérieure à la durée de vie de la bioprothèse, le recours à l'implantation d'une bioprothèse présente des risques accrus (Schoen, et al. (1999) Founder's Award, 25th Annual Meeting of the Society for Biomaterials, perspectives. Providence, RI, April 28-May 2, 1999.. J Biomed Mater Res 47, 439-465). Cela est encore plus vrai chez le sujet jeune et chez l'enfant, chez lesquels la dégradation de la bioprothèse est particulièrement accélérée (Williams, et al. (1982. J Thorac Cardiovasc Surg 84, 446-450 ; Rocchini et al. (1981) Circulation 64, II162-171). Chez le nouveau né, la bioprothèse s'altère en moins de 2 ans. Il n'existe malheureusement pas actuellement d'implant idéalement adapté chez l'enfant.

Les différents facteurs qui sont impliqués dans la calcification des bioprothèses ne sont pas tous établis. Les facteurs incriminés sont : (i) l'âge du patient au moment .de l'implantation (Bortolotti, et al. (1991). J Card Surg 6, 638-643; Jamieson, et al. (1988). Ann Thorac Surg 46, 155-162), (ii) l'existence de problèmes métaboliques (tels que hypercalcémie, affection hyperthyroïdienne, diabète, maladie de Paget, etc.), l'administration parentérale de calcium, l'insuffisance rénale chronique(Schoen, et al. (1988) J Biomed Mater Res 22, 11-36), (iii) des facteurs alimentaires, (iv) la présence d'une infection, (v) la déshydratation du tissu au moment de la pose, (vi) le stress mécanique avec notamment des distorsions à l'implantation ou de petit anneau aortique, (vii) la localisation du site d'implantation (aorte ou mitrale) (Jamieson, et al. (1995) Ann Thorac Surg 60, S235-240) (site défavorable à l'implantation en position mitrale avec contraintes mécaniques plus importantes et régime de pression systolique pendant la contraction ventriculaire alors qu'au niveau aortique la fermeture des sigmoïdes se fait en diastole pendant la relaxation cardiaque), (viii) l'infection, (ix) l'inflammation chronique (la calcification étant l'évolution classique de tous processus inflammatoire dans l'organisme (cf. Tuberculose, silicose etc...), (x) la grossesse (Jamieson, et al. (1995) Ann Thorac Surg 60, S282-286; discussion S287), (xi) la phase de croissance chez l'enfant (Silver, et al. (1980) Am J Cardiol 45, 685-689), et (xii) l'anti-coagulation initiale non optimale.

Chez le jeune homme et l'enfant, il est possible qu'un métabolisme phosphocalcique plus important que le sujet plus âgé soit à l'origine de la calcification particulièrement accéléré des bioprothèses. Cependant, Simionescu et al. (Simionescu, et al. (2004) Expert Opin Biol Ther 4, 1971-1985) ont fait une analyse des nombreux travaux scientifiques relatifs aux calcifications des bioprothèses chez l'enfant en fonction de l'adolescence et la croissance. Il apparaît que l'altération des bioprothèses a lieu de manière identique, avant ou après la phase de croissance, ce qui suggère que d'autres mécanismes que le simple métabolisme phosphocalcique sont susceptibles d'être impliqués.

Différentes techniques ont été utilisées afin de surmonter les inconvénients liés à la durée de vie limitée des bioprothèses.

On a notamment tenté de réduire les contraintes mécaniques subies par les tissus fixés de la bioprothèse, et à améliorer les techniques d'implantation, de manière à éviter de provoquer des distorsions de la bioprothèses au moment de son implantation.

D'autres techniques ont consisté à améliorer les qualités mécaniques et/ou chimiques des bioprothèses, en perfectionnant notamment les divers traitements de fixation du tissu animal de départ. Différentes méthodes ont été proposées pour améliorer la technique classique de fixation par le glutaraldehyde. On a proposé par exemple une technique de post-fixation par une solution contenant un mélange de Alcool/Tween/Formol, cette technique étant connue sous le nom de procédure de stérilisation (« sterilization procedure ») (Carpentier, et al. (1984) Circulation*).* On a aussi proposé le traitement du tissu fixé au glutaraldehyde par un agent surfactant sans agent dénaturant (Demande de brevet US 2004/0093674): Plus récemment on a aussi mis au point des techniques combinant un traitement par fixation au glutaraldehyde, un traitement adjuvant par un surfactant ou agent dénaturant et un traitement physique par la chaleur, pendant les étapes de fixation (Demandes de brevet US 2006/0217805; US 2005/0071926 ; US 2004/0030405 ; US 2003/0226208).

Pour limiter la calcification des bioprothèses, ont aussi été décrits des traitements avec des agents réticulants, autres que le glutaraldehyde (Ogle, et al. (2003) Ann Thorac Surg 75, 1267-1273; Chen, (1994) Circulation 90, 323-329; Vyavahare et al. (1997) Circulation 95, 479-488 ; Clark, et al. (2005) Ann Thorac Surg 79, 897-904).

On a également mis en oeuvre des techniques permettant l'obtention de bioprothèse ayant une géométrie améliorée afin de réduire les contraintes mécaniques subies dans le corps des patients. Egalement, pour les bioprothèses valvulaires cardiaques, on a mis au point des bioprothèses sans « stent », dans l'objectif de limiter les gradients de contraintes mécaniques au niveau des commissures (Hopkins, (2006) Circulation 114, 261-264 ; Schoen, et al. (1999) Founder's Award, 25th Annual Meeting of the Society for Biomaterials, perspectives. Providence, RI, April 28-May 2, 1999. J Biomed Mater Res 47, 439-46).

Le document Balch et al. : « Blood Group Compatibility And Aortic Valve Allo Transplantation In Man » Journal of Thoracic and Cardiovascular Surgery, Vol. 70, no 2, 1975, pages 256-259, XP009133707, ISSN :0022-5223 ) consiste en une étude académique ancienne mais qui implique un nombre très important de patient n=385 concernant la compatibilité de groupe sanguin dans l'allotransplantation de valves aortiques chez l'homme, et concerne donc la transplantation de tissus vivants et l'utilisation de tissus non fixés dans le cadre d'une allogreffe.

L'exposé qui précède illustre le fait que de nombreuses techniques ont déjà été mises au point afin d'accroître la durée de vie des bioprothèses dans le corps du patient, y compris des techniques visant à réduire ou retarder la calcification du tissu bioprothétique.

Néanmoins, il existe toujours un besoin dans l'état de la technique pour la disponibilité de bioprothèses possédant une longévité de fonctionnement accrue dans le corps des patients, en particulier de bioprothèses possédant des propriétés, réduites de calcification, tout spécialement pour les bioprothèses destinées à être implantées dans le corps de patients jeunes.

### RESUME DE L'INVENTION

En particulier, la présente invention vise à remédier à ces inconvénients, en fournissant un procédé d'obtention d'une bioprothèse médicale fixée chimiquement implantable chez un patient humain comprenant des substances qui ont été prélevées de tissu animal non-humain, permettant une longévité de fonctionnement accrue dans le corps des patients, en particulier en utilisant une bioprothèse ayant des propriétés réduites de calcification.

Ces buts sont atteints par un procédé d'obtention d'une bioprothèse médicale fixée chimiquement implantable chez un patient humain ayant les caractéristiques de la revendication 1.

Des formes de réalisation préférées du procédé d'obtention d'une bioprothèse médicale de l'invention sont revendiquées dans les revendications dépendantes.

La présente invention concerne un procédé d'obtention d'une bioprothèse médicale implantable comprenant des substances provenant de tissu animal non-humain, comprenant une étape au cours de laquelle on sélectionne positivement une bioprothèse fixée chimiquement pour son implantation dans le corps dudit patient lorsque (i) le type phénotypique dans le système ABO/ABH de ladite bioprothèsé est compatible avec (ii) le type phénotypique dans le système ABO/ABH dudit patient.

La présente invention concerne aussi un procédé d'obtention d'une bioprothèse médicale implantable comprenant des substances provenant de tissu animal, comprenant les étapes suivantes :
a) déterminer le type phénotypique dans le système ABO/ABH d'un patient dans le corps duquel une bioprothèse doit être implantée,
b) déterminer le type phénotypique dans le système ABO/ABH d'une bioprothèse candidate, ou dans une pluralité de bioprothèses candidates, et
c) sélectionner positivement une bioprothèse pour son implantation dans le corps dudit patient lorsque (i) le type phénotypique dans le système ABO/ABH de ladite bioprothèse est compatible avec (ii) le type phénotypique dans le système ABO/ABH dudit patient,
l'ordre d'exécution des étapes a) et b) étant indifférent.

L'invention est aussi relative à un procédé d'obtention d'une bioprothèse médicale implantable comprenant des substances provenant de tissu animal comprenant les étapes suivantes :
a) fournir une pluralité de bioprothèses dont le type phénotypique dans le système ABO/ABH est connu, et
b) sélectionner positivement, au sein de la pluralité desdites bioprothèses, au moins une bioprothèse pour son implantation dans le corps dudit patient lorsque (i) le type phénotypique dans le système ABO/ABH de ladite bioprothèse est compatible avec (ii) le type phénotypique dans le système ABO/ABH dudit patient.

De manière générale, une bioprothèse obtenue selon un procédé tel que défini ci-dessus possède des propriétés de calcification réduites dans le corps d'un patient dans lequel celle-ci doit être implantée.

Dans certains modes de réalisation desdites bioprothèses, les substances provenant de tissu animal contenues dans une bioprothèse sont choisies parmi des substances provenant de mammifère, de préférence un mammifère choisi parmi un porc, un bovin, un ovin, un équidé, un chameau, un phoque et un kangourou.

Lesdites bioprothèses peuvent notamment être choisies parmi les bioprothèses du type valve cardiaque, valves stentés, n'importe quel tissu cardiaque en incluant les feuillets valvulaires, patch péricardiques, systèmes de contention ventriculaire, greffons coronaires, prothèses vasculaires avec ou sans stent, shunts veineux centraux, greffons vasculaires hybrides ou pas anneaux valvulaires, des tissus souples comme tube digestif, de la peau, la vessie, des conduits vasculaires, des conduits ou tissus enroulés, filtres de veine cave inférieure, accès pour hémodlatyse, système de drainage pour glaucome oculaire, stents ou tubes endotrachéos-bronchiques, implants péniens, implants orthopédiques, implants dentaires, dispositifs maxillo-faciales de reconstruction, prothèses tendineuses, prothèses ligamentaires, tubes de régénération nerveuse, patchs, tissus reconstitués, dispositifs de délivrance d'agents actifs (comme décrit dans la demande de brevet PCT/FR2008000785), les bioprothèses artérielles, les bioprothèses vasculaires, les bioprothèses pulmonaires, les tissus de remplacement ou de régénération.

Lesdites bioprothèses peuvent notamment consister en des valves cardiaques choisies parmi les valvules mitrale, aortique, pulmonaire et tricuspide.

Dans des modes de réalisation préférés d'un procédé d'obtention de bioprothèses médicales conforme à l'invention, ledit procédé peut être en outre caractérisé en ce que la sélection d'une bioprothèsè est réalisée selon les règles de compatibilité suivantes :
- pour les patients dont le phénotype ABO/ABH est le phénotype A, on sélectionne positivement, une bioprothèse, lorsque le type phénotypique ABO/ABH de ladite bioprothèse est un phénotype choisi parmi A ou H,
- pour les patients dont le phénotype ABO/ABH est le phénotype O, on sélectionne positivement une bioprothèse, lorsque le type phénotypique ABO/ABH de ladite bioprothèse est le phénotype H,
- pour les patients dont le phénotype ABO/ABH est le phénotype B, on sélectionne positivement une bioprothèse, lorsque le type phénotypique ABO/ABH de ladite bioprothèse est un phénotype choisi parmi B (humain) ou H,
- pour les patients dont le phénotype ABO/ABH est le phénotype AB, on sélectionne positivement une bioprothèse, lorsque le type phénotypique ABO/ABH de ladite bioprothèse est un phénotype choisi parmi A, B ou H, et
- pour la totalité des patients, on sélectionne positivement une bioprothèse, lorsque le type phénotypique ABO/ABH de ladite bioprothèse n'est pas détectable.

Dans certains modes de réalisation, ledit procédé est caractérisé en outre en ce que,
- le phénotype dans le système Rhésus, respectivement (i) pour la ou les bioprothèse(s) candidate(s) et (ii) pour le patient, est déterminé ou connu, et
- une bioprothèse est sélectionnée positivement lorsqu'ést établie de plus une compatibilité pour le type phénotypique dans le système Rhésus.

Dans d'autres modes de réalisation, ledit procédé est caractérisé en ce que,
- le phénotype dans le système Lewis ou sécréteur, respectivement (i) pour la ou les bioprothèse(s) candidate(s) et (ii) pour le patient, est déterminé ou connu, et
- une bioprothèse est sélectionnée positivement lorsqu'est établie une comptabilité pour le type phénotypique dans le système Lewis.

Dans le phénotype du système Lewis, on peut attribuer les bioprothèses de phénotype le^{x-}/le^{y-} selon les memes critères de sélection que pour le phénotype A ;
on peut attribuer les bioprothèses de phénotype le^{x}-/le^{y+} selon les memes critères de sélection que pour le phénotype H ;
on peut attribuer les bioprothèses de phénotype le^{x+}/le^{y-} selon les mêmes critères de sélection que pour le phénotype I (A-/H-/I+).

Dans certains modes de réalisation, on pourra procéder à une sélection négative en retirant de manière systématique les bioprothèses de certains phénotypes ABH, comme par exemple les bioprothèses de phénotype I (A-/H-/I+).

### DESCRIPTION DETAILLEE DE L'INVENTION

La présente invention fournit de nouveaux procédés d'obtention de bioprothèses implantables qui possèdent des propriétés de calcification réduites lorsqu'elles sont implantées dans le corps du patient, et qui ont donc des propriétés de longévité accrues.

De manière surprenante, on a montré selon l'invention que l'on pouvait réduire, ou différer dans le temps, le phénomène de calcification des bioprothèses implantables si, préalablement à l'acte chirurgical d'implantation, on procédait à la sélection d'une prothèse compatible avec le patient receveur.

Plus précisément, on a montré selon l'invention que l'on pouvait réduire le phénomène de calcification d'une bioprothèse, si ladite bioprothèse implantée dans le corps d'un patient était au préalable sélectionnée selon son type phénotypique dans le système ABO/ABH, afin que le type phénotypique dans le système ABO/ABH de ladite bioprothèse soit compatible avec le type phénotypique dans le système ABO/ABH dudit patient.

On a en particulier montré dans les exemples que, après une durée donnée d'implantation *in vivo,* une première bioprothèse d'un type phénotypique ABO/ABH compatible avec le type phénotypique ABO/ABH du mammifère dans laquelle elle était implantée présentait une accumulation pondérale de calcium presque cinq fois moindre qu'une secondé bioprothèse de type phénotypique identique à la première, mais n'étant pas compatible avec le type phénotypique ABO/ABH du mammifère dans laquelle cette seconde bioprothèse était implantée.

On a montré dans les exemples, à partir d'une analyse statistique multivariée réalisée sur un échantillon de 920 patients ayant reçu une bioprothèse, que la compatibilité du phénotype ABO/ABH de la bioprothèse avec le phénotype ABO/ABH du patient constituait le facteur prédictif principal pour la longévité d'une bioprothèse implantée. En particulier, la même étude statistique multivariée montre que, de manière surprenante, le type de bioprothèse implantée ne constitue pas un facteur prédictif pertinent pour sa longévité dans le corps du patient.

Egalement, on a montré dans les exemples que, dans un échantillon de 920 patients chez lesquels on avait implanté une bioprothèse, et chez les patients ayant la plus forte probabilité d'avoir reçu une bioprothèse d'un type phénotypique ABO/ABH compatible avec leur propre phénotype ABO/ABH, la longévité des bioprothèses implantées était de 2,33 années supérieure à la longévité des bioprothèses implantées chez les patients ayant d'autres types phénotypiques ABO/ABH.

On a aussi montré que, dans un échantillon de 920 patients chez lesquels on avait implanté une bioprothèse, les patients ayant conservé une bioprothèse au-delà de 16 années étaient les patients ayant la plus forte probabilité d'avoir reçu une bioprothèse d'un type phénotypique ABO/ABH compatible avec leur propre type phénotypique ABO/ABH.

On a aussi montré que la compatibilité phénotypique ABO/ABH entre la bioprothèse implantée et le patient permettait de réduire, ou de différer dans le temps, la calcification des bioprothèses implantées. Ainsi, dans une étude statistique multivariée qui est présentée dans les exemples, les prothèses calcifiées étaient trouvées de manière prépondérante chez les patients ayant la plus forte probabilité d'avoir reçu une bioprothèse d'un type phénotypique ABO/ABH non compatible avec leur propre phénotype ABO/ABH.

On a montré en particulier que, dans une population de patients chez lesquels a été implantée une bioprothèse ayant eu une longévité d'une durée supérieure à seize ans, tous les patients avaient reçus une bioprothèse ayant un type phénotypique dans le système ABO/ABH compatible avec leur propre phénotype ABO/ABH. On a montré notamment que la totalité des patients ayant reçu une bioprothèse de grande longévité de type phénotypique A, présentaient eux-mêmes le type phénotypique compatible A.

Il résulte de ce qui précède qu'un accroissement de la longévité des bioprothèses dans le corps des patients peut être obtenu lorsque, préalablement à leur implantation, les bioprothèses sont sélectionnées pour leur compatibilité phénotypique dans le système ABO/ABH avec les patients qui sont destinés à les recevoir.

Ces résultats sont d'autant plus surprenants que, à la connaissance du demandeur, il n'a pas été montré dans la littérature une quelconque relation entre les aspects physiologiques du métabolisme calcique et les mécanismes immunitaires. On précise que les dysfonctionnements des bioprothèses qui surviennent avec le temps ne s'apparentent aucunement aux mécanismes de rejet de greffe d'organes vascularisés, comme en témoigne notamment la durée d'implantation précédant les dysfonctionnements, même précoces, qui ont lieu plusieurs années suivant l'implantation des bioprothèses, et pour des patients auxquels n'est administré aucun traitement médical à action immunosuppressive.

La présente invention concerne un procédé d'obtention d'une bioprothèse médicale implantable comprenant des substances provenant de tissu animal, comprenant une étape au cours de laquelle on sélectionne positivement une bioprothèse pour son implantation dans le corps dudit patient lorsque (i) le type phénotypique dans le système ABO/ABH de ladite bioprothèse est compatible avec (ii) le type phénotypique dans le système ABO/ABH dudit patient.

Le procédé ci-dessus peut être aussi désigné « premier procédé » dans la présente description.

La présente invention concerne aussi un procédé d'obtention d'une bioprothèse médicale implantable comprenant des substances provenant de tissu animal, comprenant les étapes suivantes :
a) déterminer le type phénotypique dans le système ABO/ABH d'un patient dans le corps duquel une bioprothèse doit être implantée,
b) déterminer le type phénotypique dans le système ABO/ABH d'une bioprothèse candidate, où dans une pluralité de bioprothèses candidates, et
c) sélectionner positivement une bioprothèse pour son implantation dans le corps dudit patient lorsque (i) le type phénotypique dans le système ABO/ABH de ladite bioprothèse est compatible avec (ii) le type phénotypique dans le système ABO/ABH dudit patient,
l'ordre d'exécution des étapes a) et b) étant indifférent, et la bioprothèse ainsi obtenue ayant des propriétés de calcification réduites dans le corps d'un patient dans lequel celle-ci doit être implantée

Le procédé ci-dessus peut être aussi désigné « second procédé » dans la présente description.

La présente invention est également relative à un procédé d'obtention d'une bioprothèse médicale implantable comprenant des substances provenant de tissu animal comprenant les étapes suivantes :
a) fournir une pluralité de bioprothèses dont le type phénotypique dans le système ABO/ABH est connu, et
b) sélectionner positivement, au sein de la pluralité desdites bioprothèses, au moins une bioprothèse pour son implantation dans le corps dudit patient lorsque (i) le type phénotypique dans le système ABO/ABH de ladite bioprothèse est compatible avec (ii) le type phénotypique dans le système ABO/ABH dudit patient,
la bioprothèse ainsi obtenue ayant des propriétés de calcification réduites dans le corps d'un patient dans lequel celle-ci doit être implantée

Le procédé ci-dessus peut être aussi désigné « troisième procédé » dans la présente description.

De manière générale, une bioprothèse obtenue par l'un quelconque des procédés ci-dessus possède des propriétés de calcification réduites dans le corps d'un patient dans lequel celle-ci est destinée à être implantée.

Les procédés ci-dessus peuvent être collectivement désignés « le procédé » ou « les procédés » dans la présente description. L'homme du métier comprend que les premier, deuxième et troisième procédés ci-dessus consistent en des alternatives d'un procédé général d'obtention de bioprothèses

Par « bioprothèse médicale implantable », « bioprothèse implantable » ou « bioprothèse », on entend selon l'invention une prothèse destinée à être implantée dans le corps humain ou animal, et qui est fabriquée, partiellement ou en totalité avec un tissu animal, ledit tissu animal ayant été traité chimiquement, biologiquement ou physiquement, afin de lui conférer les caractéristiques physiques, chimiques ou mécaniques appropriées désirées.

Les bioprothèses englobent les bioprothèses cardiaques, qui peuvent être choisies notamment parmi les valves mitrales, les valves aortiques, les valves pulmonaires, les valves tricuspides ou encore les implants de réfection pariétale.

Les bioprothèses englobent également les bioprothèses vasculaires, qui peuvent être choisies notamment parmi les conduits aortiques ou encore les conduits pulmonaires.

Les bioprothèses englobent également les ligaments artificiels, y compris les ligaments artificiels de genou, de cheville ou d'épaule, les valves équipées ou non d'un stent, un tissu cardiaque en incluant les feuillets valvulaires, les patchs péricardiques, les systèmes de contention ventriculaire, les greffons coronaires, les prothèses vasculaires avec ou sans stent, les shunts veineux centraux, les greffons vasculaires hybrides ou non-hybrides, les anneaux valvulaires, des tissus souples comme le tube digestif, la peau, la vessie, des conduits vasculaire, des conduits ou tissus enroulés, des filtres de veine cave inférieure, des accès pour hémodialyse, les systèmes de drainage pour glaucome oculaire, les stents ou tubes endotrachéos-bronchiques, les implants péniens, les implants orthopédiques, les implants dentaires, les dispositifs maxillo-faciaux de reconstruction, les prothèses tendineuses, les prothèses ligamentaires, les tubes de régénération nerveuse, les patchs, les tissus reconstitués, les dispositifs de délivrance d'agents actifs (comme décrit par exemple dans la demande de brevet PCT/FR2008000785),

Divers types de bioprothèses qui peuvent être utilisées dans le procédé d'obtention selon l'invention, sont décrits plus loin dans la présente description, en référence en particulier aux documents de l'état de la technique qui les divulguent.

Comme le sait l'homme du métier, les « substances » provenant du tissu animal varient selon le type de bioprothèse considérée. Lesdites « substances » englobent notamment des tissus animaux eux-mêmes, y compris du tissu cardiaque, du tissu vasculaire, de la peau, des ligaments, des tendons et du tissu provenant de la sous-muqueuse digestive. Le tissu cardiaque englobe les valves cardiaques et les tissus cardiaques pariétaux. Le tissu vasculaire englobe le tissu veineux et le tissu artériel, y compris les conduits des vaisseaux, les conduits veineux et les conduits artériels.

Dans certaines bioprothèses implantables, les « substances » provenant du tissu animal englobent un ou plusieurs composants de la matrice extracellulaire, tel que le collagène.

Les animaux dont proviennent lesdites substances peuvent aussi considérablement varier selon le type dé bioprothèse considérée. En général, on utilise des substances provenant de tissu de mammifère. Le plus souvent, on utilise des substances provenant de tissu d'un mammifère choisi parmi un porc, un bovin, un ovin et un équidé. Les bioprothèses les plus courantes, en particulier les bioprothèses cardiaques, sont réalisées à partir de tissu provenant de porcins et de bovins, préférentiellement de porc et de boeuf.

Ainsi, dans un procédé d'obtention d'une bioprothèse médicale implantable conforme à l'invention, les substances provenant de tissu animal contenues dans une bioprothèse sont choisies parmi des substances provenant de mammifère, de préférence un mammifère choisi parmi un porc, un bovin, un ovin et un équidé.

Par « patient », on entend selon l'invention un humain ou un mammifère non humain, ce qui englobe notamment les chiens, les chats et les chevaux.

Par « système ABO/ABH », on entend le système de groupe sanguin désigné « ABO » et « ABH » chez l'homme et « ABH » chez les animaux, en particulier chez les porcins. Les antigènes du système ABO/ABH sont exprimés dans la majorité des tissus vivants et non seulement sur les globules rouges, comme cela sera détaillé plus loin dans la présente description. Les antigènes du système ABO/ABH peuvent aussi être sécrétés et peuvent donc être retrouvés sous forme libre circulante ou bien sous forme adsorbée sur des substances corporelles ou des membranes cellulaires. Le phénotype des tissus dans le système ABO/ABH appartient généralement à l'un des quatre, groupes majeurs parmi respectivement les groupes A, B, AB et O. Comme cela sera détaillé plus loin, certains individus peuvent avoir un autre phénotype, statistiquement mineur dans une population d'individus, le type phénotypique Bombay.

Les techniques pour déterminer le type phénotypique d'un patient, dans le système ABO/ABH sont bien connues par l'homme du métier. Par exemple, on utilise couramment des tests sérologiques dans lesquels on détermine la présence ou l'absence d'anticorps anti-A et anti-B chez le patient. On rappelle que :
- chez les individus ayant le type phénotypique A on détermine la présence d'anticorps anti-B et l'absence d'anticorps anti-A,
- chez les individus ayant le type phénotypique B, on détermine la présence d'anticorps anti-A et l'absence d'anticorps anti-B,
- chez les individus AB, on détermine l'absence à la fois d'anticorps anti-A et d'anticorps anti-B,
- chez les individus de type phénotypique O, on détermine la présence à la fois d'anticorps anti-A et d'anticorps anti-B.

De manière courante, les tests de détermination du type phénotypique d'un individu dans le système ABO/ABH sont réalisés selon des techniques d'immuno-détection bien connues de l'homme du métier, notamment :
- par détermination du type phénotypique des globules rouges du patient à tester, à l'aide d'anticorps anti-A, d'anticorps anti-B et le cas échéant aussi d'anticorps anti-H, ou
- par détermination de la présence ou l'absence d'anticorps anti-A, ou d'anticorps anti-B, à l'aide d'un support sur lequel sont immobilisés des antigènes de phénotype A ou des antigènes de phénotype B, par exemple des globules rouges de phénotype connu, ou encore des supports adaptés pour les tests immunologiques sur lesquels lesdits antigènes ont été immobilisés selon des techniques connues.

Pour déterminer le type phénotypique d'une bioprothèse médicale implantable dans le système ABO/ABH, on peut mettre en oeuvre des techniques de tests immunologiques connues en elles-mêmes, qui comprennent une étape au cours de laquelle on met en contact des anticorps anti-A ou des anticorps anti-B ou des anticorps anti-H (anti-H1, anti-H2) ou des anticorps anti-I, anti-Le^{x}/anti-Le^{y}, des lectines (entre autres lectine anti-H et/ou anti-UE1 Ulex Europaeusl, lectine anti-I comme PNA Arachis Hypogaea (ref. Oriol R. Transplant international 1994)), avec la surface de ladite bioprothèse, ou plus généralement avec le matériau provenant de tissu animal contenu dans ladite bioprothèse.

Pour déterminer le type phénotypique d'une bioprothèse médicale implantable dans le système ABO/ABH, on peut aussi mettre en oeuvre des techniques de détermination du génotype correspondant au type phénotypique, dans des réactions d'amplification de l'ADN utilisant des acides nucléiques amorces appropriés, l'ADN éventuellement amplifié étant ensuite caractérisé par détection à l'aide de sondes nucléotidiques adaptées. Ces techniques de détection génotypique sont bien connues de l'homme du métier et sont aujourd'hui couramment pratiquées dans les laboratoires d'analyse médicale, à la fois dans les hôpitaux et dans les laboratoires d'analyse de pratique privée.

Les règles de « compatibilité » respectivement du type phénotypique ABO/ABH du patient et du type phénotypique ABO/ABH d'une bioprothèse sont celles qui sont usuellement utilisées dans les techniques de détermination de compatibilité ABO/ABH en sérologie.

Comme cela est montré dans lés exemples, les bioprothèses obtenues conformément au procédé ci-dessus possèdent une longévité dans le corps des patients qui est en moyenne significativement supérieure à la longévité moyenne des prothèses qui sont couramment implantées aujourd'hui. Sans vouloir être lié par une quelconque théorie, le demandeur pense que la différence de longévité observée dans les exemples entre (i) les bioprothèses compatibles avec le patient et (ii) les bioprothèses non compatibles avec le patient est vraisemblablement bien inférieure à celle qui sera observée dans la pratique, dans le cadre d'études cliniques ciblées. Cette propriété de longévité de la bioprothèse dans le corps du patient apparaît déjà comme un facteur prédominant actuel dans le choix d'attribution des bioprothèses, qui va bien au-delà de tous les autres facteurs connus.

Grâce au procédé de l'invention, il est désormais possible de réaliser des implantations de bioprothèses en limitant, dans la population des patients traités, l'incidence des altérations précoces des bioprothèses, qui surviennent après une durée d'implantation inférieure à sept ans.

Dans le premier procédé d'obtention d'une bioprothèse médicale implantable selon l'invention, la caractéristique essentielle réside dans l'étape technique de sélection positive d'une bioprothèse lorsque (i) le type phénotypique dans le système ABO/ABH de ladite bioprothèse est compatible avec (ii) le type phénotypique dans le système ABO/ABH dudit patient.

La mise en oeuvre dudit premier procédé implique que le type phénotypique dans le système ABO/ABH dudit patient ait été déterminé au préalable.

Dans certains modes de réalisation dudit premier procédé, le type phénotypique dans le système ABO/ABH de ladite bioprothèse est également déterminé au préalable, de sorte à permettre l'étape technique de sélection positive d'une bioprothèse qui soit compatible avec le patient.

Dans d'autres modes de réalisation dudit premier procédé, on utilise des bioprothèses « universelles », pour lesquelles le type phénotypique dans le système ABO/ABH est indétectable ou bien encore pour lesquelles le type phénotypique dans le système ABO/ABH est de type O, auquel cas, l'étape technique de sélection positive comprend essentiellement le fait de déterminer que l'ensemble de bioprothèses au sein duquel la sélection doit être opérée est bien référencé comme étant de type «ABO/ABH indétectable » ou bien de type « O ».

Symétriquement, l'homme du métier comprend que l'étape technique de sélection positive implique nécessairement la réalisation d'une étape technique de sélection négative à l'égard d'une bioprothèse dont le type phénotypique dans le système ABO/ABH n'est pas compatible avec le type phénotypique dans le système ABO/ABH du patient dans le corps duquel une bioprothèse médicale doit être implantée.

On précise que les caractéristiques techniques décrites ci-dessous pour les autres procédés d'obtention d'une bioprothèse médicale sont directement transposables à la mise en oeuvre du premier procédé ci-dessus, dès lors que ces caractéristiques se rapportent à une étape de sélection positive d'une bioprothèse.

Dans le second procédé d'obtention d'une bioprothèse médicale implantable de l'invention, qui comprend trois étapes essentielles, l'ordre d'exécution des étapes a) et b) est indifférent. II suffit que, au moment de réaliser l'étape c) de sélection d'une bioprothèse compatible avec le patient à laquelle elle est destinée, on connaisse à la fois le type phénotypique dudit patient et le type phénotypique de ladite bioprothèse, dans le système ABO/ABH.

Dans certains modes de réalisation du second procédé, l'étape a) de détermination du type phénotypique du patient peut avoir été réalisée jusqu'à plusieurs années précédant l'étape b) de détermination du type phénotypique de ladite bioprothèse, ce qui ne présente pas d'inconvénient, du fait que le type phénotypique d'un mammifère humain ou non-humain reste inchangé tout au long de sa vie.

Dans certains modes de réalisation du second procédé, l'étape b) peut avoir été réalisée préalablement à l'étape a), par exemple dans les situations dans lesquelles un lot de bioprothèses, pour lesquelles on a déterminé le type phénotypique ABO/ABH, a été fabriqué à un temps déterminé, jusqu'à plusieurs mois précédant l'acte chirurgical d'implantation, et que le type phénotypique du patient receveur a été déterminé postérieurement, par exemple peu de temps avant que la décision médicale d'implantation d'une bioprothèse ait été prise.

Selon le troisième procédé d'obtention d'une bioprothèse médicale implantable défini précédemment, on fournit une pluralité de bioprothèses dont le type phénotypique dans le système ABO/ABH a été préalablement déterminé et est en conséquence connu. Selon ce troisième procédé, le type phénotypique du patient receveur, dans le système ABO/ABH, a été également déterminé à l'avance et est en conséquence également connu. L'étape essentielle du troisième procédé de l'invention consiste donc, comme pour le premier procédé ci-dessus, à sélectionner, au sein de la pluralité de bioprothèses, au moins une bioprothèse dont le type phénotypique ABO/ABH est compatible avec le type phénotypique ABO/ABH du patient receveur.

Dans certains modes de réalisation d'un procédé d'obtention d'une bioprothèse médicale implantable conforme à l'invention, le fait que le phénotype ABO/ABH de ladite bioprothèse soit déterminé, peut être matérialisé par le fait que le type phénotypique ABO/ABH de ladite bioprothèse, ou bien le type phénotypique ABO/ABH du lot de fabrication de bioprothèses auquel elle appartient, soit consigné par le fabricant. Dans certains modes de réalisation, le type phénotypique ABO/ABH d'une bioprothèse peut être consigné sur l'emballage de ladite bioprothèse, ou bien sur l'emballage d'un lot contenant une pluralité de bioprothèses du même type phénotypique ABO/ABH. Dans d'autres modes de réalisation, le type phénotypique ABO/ABH de la bioprothèse peut être consigné dans un document qui accompagne l'emballage de la bioprothèse ou bien l'emballage d'un lot comprenant une pluralité de bioprothèses. Selon encore d'autres modes de réalisation, le type phénotypique ABO/ABH de la bioprothèse est consignée dans un document localisé chez le fabricant, ou chez un intermédiaire commercial fournissant les bioprothèses. Selon ces derniers modes de réalisation, le médecin peut alors, avec la commande d'une bioprothèse, déclarer le phénotype ABO/ABH du patient au fournisseur, qui peut être le fabricant, afin que ledit fournisseur lui adresse une bioprothèse compatible avec le patient receveur, la bioprothèse compatible étant choisie par ledit fournisseur à partir des données de phénotype ABO/ABH des bioprothèses dont il a connaissance. Dans encore d'autres modes de réalisation, le praticien médical peut avoir accès de manière distante à l'information du type phénotypique ABO/ABH d'une bioprothèse, par exemple par une accès en ligne à cette information auprès du fabricant ou auprès de tout intermédiaire agréé par le fabricant.

Ce qui est décrit ci-dessus au sujet de la disponibilité de l'information du type phénotypique ABO/ABH d'une bioprothèse est généralisable à l'information du type phénotypique de ladite bioprothèse, dans d'autres systèmes de compatibilité connus, dans les modes de réalisation de l'invention dans lesquels le type phénotypique dans un ou plusieurs de ces autres systèmes de compatibilité connus constitue un critère de sélection de ladite bioprothèse.

Si un phénotype d'une colonie, d'un groupe ou de race de porcs est connu, il ne sera pas nécessaire de rechercher le phénotype de chaque bioprothèse, mais l'attribution de la bioprothèse en fonction du seul phénotype du patient suffit.

Dans certains modes de réalisation des procédés d'obtention de bioprothèses conformes à l'invention, lesdites bioprothèses sont choisies parmi les bioprothèses du type valve cardiaque les bioprothèses artérielles, les bioprothèses vasculaires, les bioprothèses pulmonaires, les tissus de remplacement ou de régénération.

Les tissus de remplacement ou de régénération englobent notamment les divers tissus de la peau, comme l'épiderme et le derme.

Dans certains modes de réalisation des procédés d'obtention de bioprothèses conformes à l'invention, lesdites bioprothèses consistent en des bioprothèses valvulaires choisies parmi les valvules mitrale, aortique, pulmonaire et tricuspide.

Dans certains modes de réalisation des procédés de l'invention, le type phénotypique ABO/ABH d'une bioprothèse est identique au type phénotypique de l'animal duquel provient les substances tissulaires contenues dans la bioprothèse.

Dans certains autres modes de réalisation des procédés de l'invention, le type phénotypique ABO/ABH d'une bioprothèse est distinct du type phénotypique de l'animal duquel provient les substances tissulaires contenues dans la bioprothèse.

Par exemple, dans le procédé de fabrication de la bioprothèse, certains traitements chimiques, biologiques ou physiques du tissu animal de départ sont susceptibles d'altérer substantiellement les antigènes ABO/ABH, qui sont des sucres, de telle sorte que les antigènes ABO/ABH initialement exprimés par le tissu animal soient devenus indétectables et que le type phénotypique ABO/ABH de ladite bioprothèse soit en conséquence déterminé comme étant de phénotype H.

Dans d'autres exemples, certains traitements chimiques, biologiques ou physiques du tissu animal de départ, durant le procédé de fabrication de la bioprothèse, ont seulement partiellement altéré les antigènes ABO/ABH, le type phénotypique ABO/ABH de ladite bioprothèse étant en conséquence finalement déterminé comme étant de phénotype H ou I.

Pour les raisons ci-dessus, il est essentiel que, dans les procédés de l'invention, ce soit le type phénotypique ABO/ABH de la bioprothèse finale, sous sa forme telle qu'elle sera implantée au patient, qui soit déterminé, et non pas seulement le type phénotypique de l'animal dont provient les substances tissulaires que ladite bioprothèse comprend.

Dans certains modes de réalisation des procédés d'obtention d'une bioprothèse médicale implantable tels que définis dans la présente description, la sélection d'une bioprothèse, à l'étape unique du premier procédé, à l'étape c) du second procédé ou encore à l'étape b) du troisième procédé, est réalisée selon les règles de compatibilité suivantes :
- pour les patients dont le phénotype ABO/ABH est le phénotype A, on sélectionne positivement une bioprothèse, lorsque le type phénotypique ABO/ABH de ladite bioprothèse est un phénotype choisi parmi A ou H,
- pour les patients dont le phénotype ABO/ABH est le phénotype O, on sélectionne positivement une prothèse, lorsque le type phénotypique ABO/ABH de ladite bioprothèse est le phénotype H,
- pour les patients dont le phénotype ABO/ABH est le phénotype B, on sélectionne positivement une prothèse, lorsque le type phénotypique ABO/ABH de ladite bioprothèse est un phénotype choisi parmi B (humain) ou H,
- pour les patients dont le phénotype ABO/ABH est le phénotype AB, on sélectionne positivement une prothèse, lorsque le type phénotypique ABO/ABH de ladite bioprothèse est un groupe choisi parmi A, B ou H, et
- pour la totalité des patients, on sélectionne positivement une prothèse, lorsque le type phénotypique ABO/ABH de ladite bioprothèse n'est pas détectable.

Selon un premier aspect des modes de réalisation ci-dessus, les procédés de l'invention sont caractérisés en ce que les bioprothèses pour lesquelles le type phénotypique ABO/ABH n'est pas détectable sont choisies parmi (i) les bioprothèses comprenant des substances provenant d'un animal n'exprimant pas d'antigène du système ABO/ABH, y compris un animal génétiquement modifié et (ii) les bioprothèses ayant reçu un ou plusieurs traitements chimiques, biologique, physique ou enzymatique ayant provoqué une altération des antigènes du système ABO/ABH.

Les animaux n'exprimant pas d'antigène du système ABO/ABH sont préférentiellement les animaux qui ont été génétiquement modifiés, en particulier les animaux dont l'ADN génomique a été artificiellement modifié par les techniques de recombinaison génétique, au niveau des gènes codant les enzymes catalysant la synthèse des sucres constitutifs des antigènes du système ABO/ABH. Des techniques d'obtention d'animaux génétiquement modifiés de ce type sont par exemple décrites dans le brevet US 7,126,039, mais pour une application à la fourniture d'organes vascularisés animaux pour une implantation chez l'homme sans induire le rejet vasculaire hyper aigu du greffon hétérologue.

Les animaux génétiquement modifiés englobent les animaux dits « knock-out » pour les gènes impliqués dans la synthèse des antigènes ABO/ABH. Cela peut etre des animaux transgéniques pour le système ABO/ABH humain, notamment pour le gène codant l'enzyme, l'enzyme fucosyltransférase, ou encore une glycosyltransférase, telle que la Glycosyltransférase A1 et la Glycosyltransférase B. Ces animaux génétiquement modifés peuvent aussi etre knock-out pour d'autres résidus sucré ou d'autres antigène impliqués dans le rejet pour l'enzyme alfa-Galactosyltransférase, enzyme majeure du rejet hyperaigu xénogénique.

Dans certains modes de réalisation des procédés d'obtention d'une bioprothèse médicale implantable conformes à l'invention, les règles de compatibilité de la bioprothèse au patient receveur peuvent encore être affinées en ajoutant une exigence de compatibilité également dans le système sérologique Rhésus bien connu de l'homme du métier.

Ainsi, dans certains modes de réalisation des procédés de l'invention, ces procédés sont en outre caractérisés en ce que,
- le phénotype dans le système Rhésus, respectivement (i) pour la ou les bioprothèse(s) candidate(s) et (ii) pour le patient, est déterminé ou connu, et
- une bioprothèse est sélectionnée positivement lorsqu'est établie de plus une compatibilité pour le type phénotypique dans le système Rhésus.

Pour le type phénotypique dans le système Rhésus, on sélectionnera positivement une bioprothèse de type phénotypique Rhésus positif, lorsque le patient receveur est de type phénotypique Rhésus positif.

Pour le type phénotypique dans le système Rhésus, on sélectionnera positivement une bioprothèse de type phénotypique Rhésus négatif, lorsque le patient receveur est de type phénotypique Rhésus positif ou Rhésus négatif.

A titre illustratif, des exemples spécifiques de règles de compatibilité entre la bioprothèse et le patient receveur sont décrites ci-après..

Pour les patients du groupe O, on évitera les phénotypes de bioprothèses de type A et aussi de préférence I. On choisira de préférence les bioprothèses de phénotype A-/H+/I- en sélectionnant en deuxième possibilité, faute de mieux A-/H+/I+. On pourra donc aux patients du groupe O proposer des bioprothèses de phénotype H comme décrit précédemment.

Pour les patients du groupe B, on évitera les bioprothèses de phénotype A ou I. On choisira donc des bioprothèses de phénotype A-/H+/I- ou en deuxième intention A-/H+/I+. On pourra donc aux patients du groupe B proposer des bioprothèses de phénotype H ou phénotype B humain comme décrit précédemment

Les patients du groupe AB peuvent recevoir des bioprothèses de phénotype H , A animal ou humain ou B humain.

Pour les patients du groupe Bombay, on évitera les bioprothèses de phénotypes H, B et AB. Ces patients pourront bénéficier de bioprothèses sur lesquelles on aura diminué de manière globale l'expression des résidus sucrés.

De manière générale on évitera les bioprothèses de phénotype I associé.
Il y a deux types de bioprothèses de groupe I : les bioprothèses A-/H-/I+ et les bioprothèses ou l'antigène I est soit associé à l'antigène A soit à l'antigène H. Il est possible de distinguer ces deux groupes du fait de la différence de sensibilité pour la détection de l'antigène I entre l'anticorps anti-I et la lectine PNA. L'anticorps anti-I reconnaît l'anticorps anti-I dans les groupes A, H ou I alors que dans la majorité des cas, la lectine PNA ne reconnaît pas l'antigène I lorsque les spécificités A ou H sont exprimées. Il est donc possible d'éliminer les phénotypes A-/H-I+ biocompatibles lorsque le phénotype est reconnu à la fois par la lectine et l'anti-I.

Dans encore d'autres modes de réalisation des procédés de l'invention, ces procédés sont caractérisés en ce que,
- le phénotype dans le système Lewis, respectivement (i) pour la ou les bioprothèse(s) candidate(s) et (ii) pour le patient, est déterminé ou connu, et
- une bioprothèse est sélectionnée positivement lorsqu'est établie une comptabilité pour le type phénotypique dans le système Lewis.

Les phénotypes Lewis et Sécréteur des patients est corrélé à la présence d'un gène FUT2 qui est fonctionnel. Ce gène code également pour le core H de type 2. Il pourra être intéressant également de proposer en fonction de spécificité Lewis ou Sécrétrice des patients des porcs plutôt de type H 1 ou plutôt de type H2.
Il existe souvent une corrélation directe entre le phénotype A, H et I des bioprothèses et un autre antigène du système ABH comme par exemple dans le système Lewis. Les animaux de phénotype A+ (A+/H+/I- ou A+/H-/I+) sont généralement de phénotype Ie^{x-}/Ie^{y-}. Les animaux de phénotype H (H+/A-/I+ ou H+/A-/I-) sont généralement de phénotype Ie^{y+}/Ie^{x-}. Les animaux de phénotype I+ (A-/H-/I+). sont généralement Ie^{x+}/Ie^{y-}. Donc quand on attribue une bioprothèse de phénotype :
- A à un patient, cela revient à lui attribuer une bioprothèse de type Ie^{x-}/Ie^{y-}
- H à un patient, cela revient à lui attribuer une bioprothèse de type Ie^{y+}/Ie^{x-}.
- I à un patient, cela revient à lui attribuer une bioprothèse de type Ie^{x+}/Ie^{y-}_{.}
Ainsi, il est dans certains cas possible, dans l'attribution, de remplacer un système du groupe ABO/ABH par une combinaison d'un autre groupe du système ABO/ABH ou toute variable biologique reliée à ce système. A noter que les animaux qui ont l'antigène H+ n'ont généralement pas l'antigène I- (dans environ 80% des cas).

Dans certains modes additionnels de réalisation des procédés d'obtention d'une bioprothèse médicale implantable, conformes à l'invention, on sélectionne positivement ou négativement une bioprothèse qui est, en plus de sa compatibilité dans le système ABO/ABH, également compatible avec le type phénotypique du patient dans d'autres systèmes de compatibilité, ces autres systèmes de compatibilité englobant les systèmes MNS, P, Lutheran, Kell, Duffy, Kidd, Diégo, Cartwright, Xg, Scianna, Dombrock, Colton, Lansteiner-Wiener, Chido/Rodgers, Hh, Kx, Gerbich, Cromer, Knops, Indian, OK, RAPH, John Milton Hagen, li, Globoside et GIL. (cf. Hosoi, E. (2008) Biological and clinical aspects of ABO blood group system. J Med Invest 55, 174-182)

Dans certains autres modes additionnels de réalisation des procédés d'obtention d'une bioprothèse médicale implantable, conformes à l'invention, on sélectionne positivement une bioprothèse qui est, en plus de sa compatibilité dans le système ABO/ABH, également compatible avec le type phénotypique du patient dans d'autres systèmes dé compatibilité, ces autres systèmes de compatibilité englobant les systèmes Rhésus (CED)(gènes RHD, CE)(41), type de core H pour les groupes A, B, O ou AB par exemple (type H1, H2, H3, H4)(gènes FUT), Sécréteur (SE, se)(sécréteur/non sécréteur), Lewis (Lea, Leb, Lex) (gènes FUT), Kell (gène KEL), Kidd (gène JK), Lutheran (gène LU), MNS, Duffy, Tn, T, Cad/sd, diego(DI)(gène AE1), Cartwright (YT)(gène ACHE), Xg (XG)(gène XG), Scianna (SC)(gène SC), Dombrock (DO)(Gène DO), Colton (CO)(gène AQP1), LW (LW)(gène LW), Chido/Rodgers (CH/RG)(gène CH/RG), Kx (XK)(gène XK), Gerbich (GE)(gène GYPC), Cromer (CROM)(gène DAF), Knops (KN)(gène CR1), Indian (IN)(gène CD44); MN(glycophorine) Pk, etc... pour revue E. Hosoi (2008)

D'autres caractéristiques de l'invention sont détaillées ci-dessous, qui peuvent être prises isolément ou en combinaison, selon les modes de réalisation considérés.

### Description détaillée des systèmes de compatibilité

Les antigènes de groupes sanguins sont des résidus sucrés qui sont exprimés à la surface des globules rouges. Tous les antigènes érythrocytaires ne sont pas forcement synthétisés par les erythroblastes. Les antigènes Lewis par exemple sont adsorbés sur les hématies à partir de glycolipides transportés dans le plasma. Les antigènes les plus connus sont les antigènes ABO_et rhésus mais il existe d'autres antigènes de groupes sanguins comme les antigènes Lewis, Kell, Duffy, Tn, T, Cad/sd, Pk, P etc... pour revue E. Hosoi (29) 2008. Jusqu'à récemment, la reconnaissance du groupe ABO chez l'homme se faisait par la capacité des anticorps de leur sérum à agglutiner des hématies ayant des antigènes spécifiques. Ainsi, chez l'homme, en fonction de son groupe sanguin ABO, il existe également dans son sérum des anticorps naturels dirigés contre des antigènes A chez les personnes du groupe O ou B, contre les antigènes B chez les personnes du groupe A ou O et enfin l'absence de ces anticorps chez les personnes du groupe AB. En fait il existe plusieurs groupes A1, A2, B, O1, 02, AB. Entre les groupes A1 et A2 le sucre reconnu est le même. Il s'agit plus d'un niveau d'expression et de localisation de l'antigène dans les tissus qui est différente (30). Du fait de la complexité des groupes ABO, les techniques de biologie moléculaire (31-34) risquent dans quelques années remplacer les technique actuelles par sérologie. En fait le système ABO chez l'homme est un système complexe qui inclut d'autres spécificités comme entre autres les spécificités Rhesus (85% sont Rhesus + (DD ou Dd), Lewis + (Le (a+b-) ou Le (a-b+)), Duffy, Kell, Kidd, Lutheran, MNS et sécréteur. En fonction ou pas de la présence de l'antigène, les individus ont ou pas dans leur sang des anticorps vis-à-vis de l'antigène manquant. Le titre de ces anticorps peut augmenter après transfusion ou grossesse. L'incidence des différents groupes dépend de l'origine ethnique. Dans la population caucasienne la fréquence des groupes A et O est supérieure à 40-45% chacune, la fréquence des groupes B est inférieure à 10%, et celle des groupes AB au alentour de 3%. Le groupe B en Asie représente 20% de la population et 15-20% en Afrique. Le groupe AB représente 10-15% de la population en Asie.

L'antigène ABO n'est pas uniquement un antigène exprimé sur les globules rouges. Le groupe ABO peut être présent chez les patients dans les secrétions comme les secrétions salivaires, ou le lait sous forme de mucine. Il est retrouvé sur différents facteurs circulants comme sur des protéines de la coagulation (Facteur de Willebrands). Il est également exprimé sur de nombreux tissus, notamment les tissus glandulaires, au niveau des cellules endothéliales et épithéliales. En fait différents facteurs influencent le type, la quantité et la distribution histologique des groupes sanguins dans les tissus, notamment la spécificité ABO, Lewis, spécificité sécréteur ou pas, le type de cellules et le type de tissu (35). Chez les patients dits « sécréteurs » le groupe ABO est exprimé également dans la salive ou d'autres sécrétions. Ils peuvent être également exprimés dans les phanères comme les poils. Au galactose de l'antigène H, un galactose est associé sous la forme d'une liaison alpha 1,3 pour former le groupe B antigène (trisaccharide). Si un N-acétylgalactosamine est transféré sur le galactose de l'antigène H le groupe A trisacharide antigène est formé. La formation des groupes A et B est sous la dépendance d'une enzyme glycosyl transférase A ou B codée par les gènes A et B. Pour les individus du groupe O ces enzymes ne sont pas actives. Ils expriment chez ces individus une enzyme spécifique codant pour les spécificités 01 ou 02 mais cette enzyme n'est, pas active du fait d'une délétion. Il existe également deux types de gènes A1 et A2 qui codent pour une galactosyl A transférase de plus ou moins grande activité avec une plus ou moins grande expression tissulaire de l'antigène A. Différents core H on été décrit, au moins 4 chez l'homme. Les plus communs sont les types 1 et 2. Le types 3 et 4 se trouvent surtout dans l'appareil gastro-intestinal et au niveau de l'épithélium respiratoire. Chez l'homme comme chez le porc, le groupe H des cellules épithéliales de l'estomac, les substances H, sont des structures hydrocarbonées portées par des protéines « O-glycozylated linked » par pont GalNAc à des Ser or Thr sur une protéine de surface (36).

D'autres antigènes sont reliés au groupe ABO comme les antigènes rhésus et Lewis, Kell, Duffy. La spécificité Lewis est formée sous l'action d'une fucosyl transférase. En fait les spécificités H, sécréteurs et Lewis sont déterminées par l'addition de L-fucose sur deux substrats principaux dont le motif caractéristique présent à l'extrémité des chaînes glycanniques de divers glycoconjugués est un disaccharide de type 1 (Galβ1-3GlcNAc) ou de type 2 (Galβ1-4GlcNAc) respectivement. La fucosyltransférase H catalyse le transfert du L-fucose (sous forme de GDPFuc) en position α1-2 sur le Galactose (Gal) des chaînes de type 2 principalement tandis que la fucosyltransférase participant au phénotype sécrétoire SE du patient transfère le L-fucose en même position sur les chaînes de type 1 principalement. La fucosyltransférase participant à l'élaboration de la spécificité Lewis transfère le L-Fucose d'une part en position α1-4 sur le N-acétylglucosamine (GlAc) des chaînes de type 1 et H de type 1 pour former les produits Le^{a} et Le^{b} respectivement et d'autre part en position α1-3 sur les chaînes de type 2 et H de type 2 pour former les produits connus sous différents nomenclatures : (CD15, X, Le^{x}) ou (SSEA-1 et Y ou Le^{y}).

Les fucosyl transférases sont en fait codées par des gènes FUT (pour revue «JP Baron Vers une approche moléculaire de la structure, du polymorphisme et de la fonction des groupes sanguins TCB (1996)181-210 »). La spécificité Lewis est en fait codée par une fucosyl transférase FUT 4-6 notamment FUT3. D'autres activités sont codées par des fucosyl transférases comme le type sécréteur ou pas déterminé par une autre fucosyl transférase, fucosyl 2 codée par les gènes FUT2. La formation du groupe H type 1 implique surtout FUT2 et un peu FUT1. La formation du groupe H de type 2 implique surtout FUT1 et peu FUT2. H type 1 : Galβ1-3GlcNAc-R avec un L-Fuc on α1-2 of Gal /H type 2 : Galβ1-4GlcNAc-R avec un L-Fuc on α1-2 of Gal.

Les antigènes Lewis peuvent être « sialylated » notamment dans des phénomènes tissulaires tumoraux ou inflammatoires chroniques. L'antigène Le^{a} est codé par FUT3. Profil sécréteur SE par FUT2. Le^{x}, CD15 (X, SSEA-1) par FUT4-6 mais aussi FUT3 (LE). Le^{b} par FUT3 et FUT2 ou possiblement FUT1. Cela explique pourquoi les Le^{b} sont forcément sécréteurs, puisque la formation de l'antigène nécessite que FUT2 gène soit actif. Slex Sylil-type 2 implique FUT6 ; FUT7 ou FUT5. Chez l'animal comme le porc, les animaux de phénotype A+ sont généralement Ie^{x-}/Ie^{y-}. Les animaux A-/H+ sont généralement Ie^{y+}/Ie^{x-}. Les animaux A-/H-/I+ sont généralement Ie^{x+}/Ie^{y-}.

L'exposé ci-dessus illustre comment le type de chaîne H présent chez un homme ou un animal peut être intiment lié à son phénotype sécréteur ou non-sécréteur, dans le système Lewis. Chez l'animal il a été démontré que la susceptibilité à certaines infections ou certains comportements étaient corrélés également à l'expression de certaines formes de fucosyl transférase.

### Bioprothèses médicales implantables utilisables dans les procédés de l'invention

Les bioprothèses médicales implantables englobent tout dispositif destiné à être implanté chez l'homme et qui contient en partie ou en totalité du tissu animal ou des produits de synthèse animale, ou des composants purifiés à partir d'un tissu animal et réticulé et/ou fixé. Le dispositif peut contenir d'autres composants biologiques autologues, homologues, synthétisés ou synthétiques. Le dispositif peut être un tissus (par exemple US Patent 6936070, 5067962, 6790213, 4585458, 7404819, 20070254005), une matrice (par exemple US Patent 20010051824, 20040157206, 6652583, 6174333, 5855620, 5613982). du collagène. (par exemple US Patent 20030203008, 6548077, 6127143, 5814328, 5374539), un conduit, une bioprothèse cardiaque (par exemple 20090118826, 7348175, 20020173843, 5824061, 6391538, 7316712, 20090030511, 6719789, 6074417, 7011681, 6530952, 5824067, 20040024452, 5769780, 4692164, 4626255, 20080154358, 2003010729 7331993, 7503930, 7503929, 6997950, 20030196274, 20030181974, 20030181974, 7322932, 6027530, 7166124, 7163556, 6540781, 20010002445, 7354749, 20080095662, 5545214, 20040106991, 7320705, 2004143323, 7455689, 5662704, 20030125805, 20030125793, 7125418, 7125418, 7318998, 7041132, 7033390, 6719785, 6682558, 6087552, 5755782, 5571174, 5549665, 5545215, 5489297, 5352240, 5326370, 5728152, 5156621, 5080670, 4626255, 4561129, 4388735, 4378224, 2008702554, 7579381, 7214344, 6878168, 6561970, 6547827, 6214054, 6008292, 5935168, 5931969, 5931969, 5782931, 5215541, 4885005, 4838888, 4648881, 4647283, 20060217805, 20040052830, 20030228692, 5632778, 5613982, 6350732, 20040136965, 7129035, 7014655, 6861211, 7438850, 6203755, 20060207031, 20050071926, 20040253291, 20050010284, 6322593, 6302909, 6231614, 6193749, 6177514, 6156531, 6156531, 6132986, 6093530, 5919472, 5094661, 5002566 , 4976733, 5447536, 5368608, 7479164, 5733339, 6596471, 30030196274, 7156881, 20020091445, 6998418, 6545042, 7014655, 6106555, 5080670, 7078163, 6509145, 2003010746, 5935168, 6471723, 6350732, 5613982), une valve cardiaque, un patch (par exemple US Patent 20010051824, 20040157206, 6652583, 6174333, 5855620), une prothèse valvulaire (par exemple US patent 20090118826; 5545215, WO/2000/047136), un échafaudage comme défini par exemple dans PCT/FR2008000785. Il peut être injectable. Il peut s'agir d'un support dont la polymérisation de quelques composants se produit spontanément ou après implantation ou après photo-activation, irradiation, par ultraviolet, irradiation gamma, courant électrique, interaction magnétique, interaction ionique, chimique, enzymatique, biologique, température, ultrasons, sel, enroulements hydrophobes/hydrophiles, force de van der Waals, liaison aromatique π métal ligand, pH, concentration, redox, phosphorylation, empilement, forces mécaniques, électromagnétiques ou gravitationnelles ou association.

Au sens de l'invention, les bioprothèses médicales implantables englobent tout tissu hétérologue fixé. Tissu / extrait tissulaire / collagène : les termes tissus ou extrait tissulaire ou collagène peuvent être employés l'un pour l'autre et associés. Il peut être naturel ou synthétique. Ce tissu peut être dé-cellularisé ou pas physiquement et/ou enzymatiquement (par exemple avec la collagénase) et/ou chimiquement modifié ou associé (comme par exemple US patent 20040052830, 20030228692, 5632778, 5613982, 20010000804, 20050266390). Ce tissu peut être compressé (comme par exemple US patent 7141064). Le tissus peut être associé à des composant synthétiques (comme par exemple US patent 20020172706, 6596024, 6562069, 4729139, 4481009). Le tissu peut être réticulé par exemple le Sulfo/NHS (20060159641, 7479164) ou par la génipine (20020091445, 6998418) par la transglutaminase. Les méthodes générales de réticulation ont été décrites (US patent 20020177223). La réticulation peut être réversible volontairement (US patent 20050244460).

Le dispositif peut être un tissu comme par exemple US Patent 7189259, une valve cardiaque ou une partie du coeur, un conduit vasculaire comme par exemple 20040158320, 20010020191, 6358275, 6206917, 6110212, 6087552), une matrice comme par exemple US Patent 20040157206, 6652583, 6174333, 5855620), du collagène (US Patent 20030203008, 6548077, 6127143, 5814328, 5374539).

Le tissu peut être fixé de manière à en améliorer les propriétés, par exemple, physique, la résistance à la dégradation, l'immunogénicité en général, sa propension à la calcification, sa thromboginicité, sa compliance, sa résistance, ses propriétés biologiques. Il peut être modifié pour en modifier son antigénicité « ABH » au sens large pour le rendre plus compatible avec un phénotype « ABO ». Par extrait de tissus sont inclus notamment les composants de la matrice extracellulaire comme les protéines dont le collagène, l'élastine. Différents mode de purification du collagènes ont été proposés comme par exemple (US Patent 20030203008, 6548077, 6127143, 5814328, 5374539).

Le collagène peut être synthétisé. Les différents composants, dont le collagène, peuvent être modifiés. Le terme collagène inclut les différents types de collagènes comme par exemple les collagènes (I, II, III, IV, V, VI, VII, XI et d'autres collagènes), ou de l'association de différentes espèces. Le terme « collagène » désigne également les collagène insoluble, collagène soluble, atélocollagène préparé en enlevant des télopeptides sur les extrémités des molécules de collagène en utilisant une protéase autre que la collagénase. Le collagène ou les tissus peuvent être préparés à partir de tissus comme uretère, péricarde, valves cardiaques, tissu digestifs comme par exemple la sous muqueuse intestinale de porc « SIS » {Lindberg, 2001 #184; Badylak, 1998 #185}, vaisseau sanguin, tendon, fascia, derme dé-cellularisé ou pas, aponévrose, membrane type membrane amniotique, dure-mère, valve cardiaque etc....). Ce pourrait être des copies synthétiques du collagène telles que des fibres de polymères ou des peptides formant des fibrilles. Le collagène peut être chimiquement modifié et le produit obtenu par succinylation ou estérification ou formation de carboxyamides, ou désamination des collagènes ci-dessus décrits, mélange de collagène avec des polymères synthétiques tels que poly-acide lactique) (PGA) et/ou poly (DL-lactide-Co-glycolide) (PLGA) et/ou poly (DL-lactide-Co-caprolactone) (PCL), un dérivé de collagène tel que la gélatine, un polypeptide obtenu par l'hydrolyse du collagène, collagène dénaturé par le chauffage. Des polymères synthétiques liés au collagène peuvent être choisis parmi l'acide polylactique (PLA), l'acide polyglycolique (PGA), poly (L-lactique) (PLLA), PLGA, poly (anhydrides) (PA), le polycarbonate (PC), les hydroxy acides, poly ortho- esters (POE), propylfumarates) (PPF), polysaccharides, la polylactone (PL), des poly caprolactones, polyamides, acides de polyamino, polyacétals des polyphosphazènes (PPZ), polycyanoacrylates biodégradables, les polyuréthanes biodégradables (unité centrale), polysaccharides, polypyrrole, polyanilines, polythiophène, polystyrène, polyester (PE), polyuréthanes non-biodégradables, polyurées, poly (éthylène-téréphtalate) (PET), poly (acétate de vinyle d'éthylène), polypropylène, polyméthacrylate, polyéthylène, polycarbonates, poly oxyde d'éthylène, poly alcool de vinyle (PVA), fuseau-tex (polytétrafluoroéthylène), dacron (téréphtalate de polyéthylène), polytétrafluoroéthylène (PTFE), polyéthylène-glycol (PEG), copolymères décrits ci-dessus, avec l'un des additifs ci-dessus, et mélanges de l'un des polymères, des copolymères, et des additifs entre eux et association de dérivés synthétiques avec les produits biologiques.

Le collagène, extrait tissulaire, peut être seulement associé à des substances synthétiques, inorganiques (comme le verre, Si/Si02, titane/oxyde de titane, or, chrome, cobalt, diamant, platine et hydroxyapaptite, nitinol, acier, silice, streptavidine-biotine, une protéine artificielle comme le latex, le nylon, le catguth, le coton, la toile, le polyester, la soie, le plastique, la céramique, les alliages, le textile, l'avidine, la streptavidine, l'éponge de copolymère - caprolactone-Co-L-lactide renforcée avec du poly-L-lactide, faite en tissu tricoté d'acide hyaluronique (PCLA), l'amidon et n'importe quel mélange), matériaux organiques biologiques (tels que protéoglycanes, glycoprotéines, glycoaminoglycanes, alginate, agarose, l'acide hyaluronique, l'agarose chitosane, le couple fibrinogène/fibrine, le chitosane carboxyméthylé et le mélange de ceux-ci, gélatine, octasulfate de sucrose, dextrane, cellulose, cellulose méthylée, sépharose, protéine imitée par le Sephadex (comme le latex) ou leur association.

Les tissus peuvent être modifiés chimiquement, enzymatiquement, ou physiquement. Il peut être associé a différentes molécules ou agents bioactifs incluant des molécules d'adhésions, ces différents agents pouvant être ou non fixés à des composants du tissus. Les agents bioactifs et molécules d'adhésions ont été définies dans la demande de brevet internationale (PCT/FR2008000785).

Dans les agents bioactifs sont inclus des stimuli physiques comme par exemple stress, chaleur, électromagnétique. Il peut être soumis à des processus de stérilisation (comme par exemple US Patent 7438850, 6203755, 2008008906, 6946098, 6908591, 6682695, 6036918) et à des traitements visant à améliorer sa préservation (comme par exemple US patent 20040136965, 7129035, 7014655, 6861211). Les différents tissus et traitement peuvent être associés.

### Méthodes d'obtention de tissu animal d'un type phénotypique désiré, pour la fabrication de bioprothèses médicales implantables

### 1) Obtention d'un tissu ABH porcin/ABO-ABH humain déterminé :

« Système ABO-ABH humain » : Par « système ABO-ABH » on entend les différents antigènes du système ABO ou ABH sanguin ou tissulaire humain au sens large ainsi que les gènes, les produits de transcription de ces gènes facteurs de régulations, sucres ou résidus sucrés ou antigènes reliés à ces antigènes et leurs régulateurs ainsi que toute variable reliée au produit d'expression de ces antigènes, groupe ABO (32, 34, 37) ou ABH/Lewis/sécréteur tissulaire (30, 38) du patient receveur (35, 39, 40). Les antigènes ABO sont des résidus sucrés portés par des protéines ou des lipides. Il s'agit de résidus sucrés glycoprotéines ou glycolipides en général. Il ne s'agit pas seulement des antigènes du système ABO sanguin, mais de la famille des ces antigènes exprimés dans le sang, mais aussi dans les tissus produits de sécrétions, liquides. Il peut s'agir du système ABO du groupe sanguin du patient au sens large avec les différents déterminants antigéniques rapportés qui ne sont pas forcément exprimés sur les globules rouges mais peuvent être exprimés dans les tissus ou dans des produits de sécrétion, salives, fluide humains. Il s'agit des groupes ABO au sens large qui contiennent plus de 23 principaux systèmes selon la nomenclature internationale de transfusion sanguine dont (cf. A (A1, A2), B, AB, O)(gène ABO) mais aussi des autres spécificités Rhésus (CED)(gènes RHD, CE)(41), type de core H pour les groupes A, B, O ou AB par exemple (type H1, H2, H3, H4)(gènes FUT), Sécréteur (SE, se)(secreteur/non secreteur), Lewis (Lea, Leb, Lex) (gènes FUT), Kell (gène KEL), Kidd (gène JK), Lutheran (gène LU), MNS, Duffy, Tn, T, Cad/sd, diego(Dl)(gène AE1), Cartwright (YT)(gène ACHE), Xg (XG)(gène XG), Scianna (SC)(gène SC), Dombrock (DO)(Gène DO), Colton (CO)(gène AQP1), LW (LW)(gène LW), Chido/Rodgers (CH/RG)(gène CH/RG), Kx (XK)(gène XK), Gerbich (GE)(gène GYPC), Cromer (CROM)(gène DAF), Knops (KN)(gène CR1), Indian (IN)(gène CD44), MN(glycophorine) Pk, etc... pour revue E. Hosoi (2008)

Il existe de très nombreuses mutations pour ces gènes. Une de ces mutations correspond à l'absence de chaîne H chez les patients Bombay/Parabombay. Il peut s'agir d'une mutation, d'une combinaison, d'une expression particulaire par exemple dans un tissu particulier ou un niveau d'expression de ces antigènes. Il peut s'agir également de pseudogène associé au système ABO par exemple «.htg4 humain pseudogene ». Le groupe ABO est représenté par les différents antigènes mais aussi par les gènes codant pour les différentes spécificités ou régulant leur expression.

Par exemple, le groupe A est principalement codé par une enzyme, l'A transférase, le groupe B par une enzyme B transférase. Il existe au moins deux gènes codant pour la A transférase A1 er A2 qui ont des niveaux d'activité différents, d'où une expression tissulaire différente de l'antigène. La spécificité H et la spécificité Lewis et Sécréteur sont sous la dépendance de gènes de type FUT (42).

Il peut s'agir également de gènes dont la synthèse implique des enzymes du système ABO. Ces mêmes gènes sont également impliqués dans la réalisation de spécificités (CD15, X, Le^{x}) ou (SSEA-1 et Y ou Le^{y}). Il peut s'agir d'antigènes n'appartement pas directement au groupe ABO mais dont la synthèse met en jeu des enzymes ou des gènes ou régulateur de gène du groupe ABO comme les sialyl Lea reconnus par l'anticorps CA19.9. (CD15, X, Le^{x}) ou (SSEA-1 et Y ou Le^{y}) ou l'antigène de Forssman glycolipid synthetase (33).

Il peut s'agir également des gènes codant pour ces différentes spécificités ou facteurs de régulation ou de modification de l'expression de ces différents antigènes ou antigènes corrélés à l'expression de certains de ces antigènes. Même facteur de transcription, même localisation sur le même gène ou très proche sur le génome.
Il peut s'agir également d'antigènes sucrés ou pas dont l'expression est corrélée à certains de ces groupes. Plus largement il peut s'agir de comportement ou de propriétés particulières biologiques ou pas de patient appartenant à un sous groupe de cette famille ABO-ABH.

### Phénotype ABH du tissu animal:

Par « phénotype ABH » du tissu on sous entend le système ABH animal (43) qui est le corolaire du système ABO chez l'homme avec une expression des antigènes dans le sang mais surtout dans les tissus ou produits de sécrétions ou liquides. Le phénotype ABH correspond à celui de la bioprothèse au moment de son implantation. La modification du phénotype ABH si nécessaire peut avoir lieu à n'importe quel moment au cours de la fabrication de la bioprothèse. Ces dérivés sucrés sont généralement exprimés sous la forme de glycosphyngolipide à la surface des cellules notamment épithéliale (44) N-linked (45) ou au niveau des mucines de sécrétion (cf. Julenius, {2005 #297}). De même, le mode de fixation des groupe H sur les cellules épithéliales chez le porc (36) ou dans les sécrétions sous forme de mucine (46) (47) a été rapportée. Si un N-acetylgalactosamine est transféré sur le galactose de l'antigène H le groupe A trissacharide antigène est formé. La formation des groupes A est sous la dépendance d'une enzyme glycosyl transférase A codée par les gènes A transférase identique au gène A humain.

L'invention propose une méthode de sélection pour un patient de groupe sanguin donné A, O, B, AB ou Lewis sanguin ou ABH tissulaire d'un phénotype particulier ABH/Lewis/sécréteur sanguin/tissulaire chez l'animal donneur (43) notamment le porc (48-51) ou le boeuf ou cheval en sachant que les antigènes ABH sont exprimés dans la majorité des espèces animales. L'expression tissulaire de certains antigènes par exemple A, H est cependant variable en fonction de l'espèce mais aussi de la race pour une espèce. Il sera dans ce cas la possible de proposer pour un patient de groupe ABO au sens large donné la préparation d'implants à partir de telle ou telle race, l'attribution se faisant en regard de l'incidence de l'expression de l'antigène désiré dans la race considérée. Il n'en reste pas moins que le choix de l'attribution de l'implant prendra en compte le groupe ABO au sens large du patient. Il est également concevable d'avoir différentes colonies ou clones d'un animal déterminé présentant les antigènes souhaités ou n'exprimant pas ces antigènes notamment au niveau ABH sanguin et tissulaire ou n'exprimant pas certains antigènes du système ABH. Là encore, l'attribution du choix de l'implant prendra en compte le système ABO sanguin tissulaire ou variables reliées du patient

Le système ABH existe donc chez la majorité des mammifères notamment chez le porc et chez le boeuf ou le cheval ou le kangourou ou le phoque: Chez le porc il existe de nombreux antigène de groupe sanguin A à L avec différents allèles de gène codant pour chacun d'eux. Ce système ABH peut être recherché de manière non exclusive dans le sang, les tissus notamment les tissus sécrétoire (tube digestif appareil urogénitale, glandes salivaires) les produits de sécrétion salives, protéines digestives, lait etc... en faisant appel à des techniques immunologiques, histologiques, ou génétiques ou combinaison. Une approche intéressante car rapide est la recherche directe du groupe ABH sur les cellules épithéliales salivaires (51). Trois types de gènes sont particulièrement exprimés : l'antigène A, l'antigène H et l'antigène I. L'antigène I est spécifique du porc. L'antigène A et H sont semblables à l'antigène A humain. L'antigène B est différent. D'après les études de Oriol (43, 49) et de (51) et (52) il existe globalement 3 phénotypes principaux A+, H+ et I+ d'incidence respective 51%, 38% et 11%. La classification se fait d'après l'expression ou pas de l'antigénicité A. Si absence d'antigénicité A présence ou pas d'antigénicité H. Les porcs A et H négatifs sont généralement de.type I.

Les porcs de phénotype A+ sont généralement le^{x}/le^{y-}. Les porcs de phénotype H+ sont généralement le^{y+}/le^{x-}. Les porcs de phénotype I+ sont généralement le^{x+}/le^{y-}. Il existe généralement, notamment dans le groupe A lorsque l'antigène H est exprimé, une absence de l'expression de l'antigène I.

Les porcs de phénotype I+ (A-/H-/I+) sont à la fois reconnus par la lectine PNA et l'anticorps anti-I. Les porcs A+ et H+ sont les porcs généralement pas reconnus par la lectine PNA. La reconnaissance de l'antigène I dans les porcs de phénotype A+ et H+ se fait généralement en utilisant un anticorps anti-I.

### Obtention de tissus animal de « phénotype A humain » :

Pour l'antigène A animal, quand il est exprimé, il existe une similitude complète avec l'antigène A humain. La spécificité A est sous la dépendance de l'enzyme A transférase. L'approche la plus facile est la sélection d'animaux exprimant cette spécificité. On peut éventuellement réaliser des élevages avec des individus ayant cette spécificité. Pour les porcs au phénotype A il existe en fait au moins deux types de porcs. Les porcs A+ et les porcs A+ et H+ qui expriment donc le résidu sucré sur un core H comme chez l'homme. Les deux types de porcs sont intéressants même si le second type est plus compatible.

L'incidence des phénotypes A dépend de la race porcine. Par exemple l'incidence du groupe A est de 23% chez les porcs Danish Landrace, 35% chez la race Hampshire et 49% dans la race Duroc. Le groupe H 46% chez les Durocs, 29% chez les Danish Landrace et 13 % chez les Hamshire. L'expression du groupe I est de l'ordre de 40-48% (*cf.* Andresen E. 1961 PNAS Pig Blood group antigènes).

Pour le groupe A, la majorité 52% n'expriment ni H ni I et sont donc A+/H-/I-. Cependant, un certain nombre des porcs de ce groupe A expriment également l'antigène H humain et sont donc très compatibles pour le groupe A (A+/H+/I-) (16%), alors que d'autres expriment l'antigène I et sont donc moins compatibles A+/H-/I+ (31 %).

Une autre approche consiste à obtenir des porcs génétiquement modifié qui expriment la spécificité A transférase humaine et utiliser des tissus à partir de ces porcs pour fabriquer des bioprothèses. Cette modification peut d'ailleurs être accompagnée d'autres modifications génétiques de ces porcs visant à inhiber notamment l'expression d'autres résidus sucrés comme les antigènes alpha-Gal : alphaGal(Galα1,3Galβ1,4GlcNAcβ1-R) (« αGal ») antigène majeur du rejet vasculaire xenogénique (KZ. Konakci et al. (53)).

### Obtention de tissus animal de « phénotype H » :

Pour l'antigène H il s'agit dans certains cas de l'antigène H humain plutôt de type 1 ou 2 selon les tissus ou l'endroit où l'on recherche l'antigène et les porcs testés. On utilise des anticorps anti H, anti-H1 ou anti-H2 ou d'autre isotypes ou des lectines comme Ulex Europaeus1, par exemple, ou par génétique. Les gènes codant les spécificités H1 et H2 sont les gènes FUT1 et FUT2 qui sont des fucosyl transférase. Le phénotype H+ représente 38% des porcs. Pour le groupe H 78% sont de vrai H (A-/H+/I-) mais 22% expriment aussi l'antigénicité I. D'autre part la spécificité H est également présente chez 16 % des porcs de groupe A (A+/H+/I-). Les porcs H+ (A-/H+) sont généralement le^{y+}/le^{x-} et I+ ou I- (généralement I-). Les porcs A+ (A+/H+) sont généralement I- également.

Différentes approches sont donc également possibles. La première consiste à sélectionner les animaux ayant un phénotype H. Il s'agit d'animaux reconnus notamment par les anticorps anti-H, soit les animaux reconnus par les lectines qui reconnaissent l'antigène H, soit les systèmes le^{x-}/le^{y+}. L'autre solution est de réaliser une sélection négative en éliminant les animaux qui n'ont pas de phénotype H dans leur population, en éliminant les tissus phénotype I+ (les tissus le^{y+}/le^{y-} ou tissus) et les tissus qui réagissent avec la lectine anti-I. On se retrouve donc avec une population enrichie en phénotype A+/H-/I-, en phénotype A+/H+/I- et en phénotype A-/H+/I-. Pour obtenir une population de phénotype A+/H- ou A+/H+, il est possible de procéder à une séléction négative d'animaux de phénotypes A-/H+ car ces animaux sont généralement de phénotype le^{y+}/le^{x-}. On pourra alors éliminer les individus qui ne réagissent pas à l'antigène H ou EUA1 et la population restante sera alors enrichie en phénotype A+/H+/I- (c'est un exemple de sélection négative pour obtenir des phénotypes cibles). Une telle population est plus « compatible » pour un phénotype de valve destinée à etre implantée chez l'homme. Une autre solution est d'utiliser des tissus préparés à partir de porc A+/H+ et de digérer ces tissus par une enzyme comme par exemple alpha-D-Galactosidase afin d'obtenir des tissus de type A-/H+. Les techniques de digestion par les exoglycosidase peuvent être aussi utilisées (54) (54).

Différentes approches sont également possibles, Comme pour le groupe A il est possible d'avoir recours à des tissus animaux transgéniques exprimant certains gènes humains codant pour le core H notamment les gènes FUT.

### Obtention de tissus animal de « phénotype B humain »:

Contrairement aux spécificités H et B il n'existe pas de porc exprimant les spécificités B humaines. Cependant chez un patient groupe B les tissus obtenus à partir d'animaux de phénotype H sont bien tolérés. Il est éventuellement possible de générer des animaux transgénique exprimant l'activité B transférase humaine.

### Non sélection de certains animaux en fonction de leur phénotype ABH :

Le phénotypage des porcs a un double intérêt. A la fois de sélectionner les porcs ayant l'antigène d'intérêt mais aussi de ne pas sélectionner les porcs dont les spécificité antigéniques ABH sont moins compatible avec l'homme. Sont concernés, notamment, les porcs de phénotype I (A-/H-/I+, A-/H^{+/-}/I+ et A-/H+/I+). L'antigène I+ peut etre assez facilement identifié en utilisant l'anticorps anti-I quel que soit le groupe A+, H+ ou I+, alors que les porcs I+ vont etre généralement des porcs PNA+ ou PNA et anti-I+.
De manière générale, on peut soit sélectionner positivement les porcs pour l'expression dés antigène A+, H+ ou I+ ou les spécificités le^{x}/le^{y} qui sont directement corrélées à l'expression de ces phénotypes. On peut effectuer une sélection négative pour augmenter dans la population la fréquence de l'antigène auquel on s'intéresse, par exemple dans le choix de la race d'origine, ou en éliminant les animaux de phénotype I+ (PNA+, anti I+), soit en éliminant les animaux exprimant certains antigènes comme l'antigène I (animaux anti-I+ et PNA+/-). Un autre mode de sélection négative pour le phénotype A, est d'éliminer les phénotypes H+ (reconnus par les anticorps H+ et lectine UEA+) et/ou I+. Cela revient aussi à éliminer les phénotypes le^{x+}/le^{y-} ou le^{x-}/le^{y+}.
On peut obtenir une population H par sélection négative en éliminant les phénotypes A+ (anti-A) et I+ (anti-I et PNA+). La population H+ peut aussi etre obtenue en éliminant les phénotypes le^{y-}. Pour le groupe I la majorité sont A+/H-/I+, A-/H+/I+ ou A-/H-/I+ : 16%.

### Obtention de tissus ayant une faible expression d'antigène ABH :

Il est possible également dans certains cas d'utiliser des moyens enzymatiques, physiques ou chimiques ou associations visant à retirer les antigènes ABH fixés sur les tissus. Cependant dans la majorité des cas cette approche va altérer les propriétés des tissus.

Les structures chimiques des cores H type 1 et 2 humains sont bien connus et généralement exprimés sous la forme de glycosphyngolipide à la surface des cellules notamment épithéliale (44) N-linked (45) ou au niveau des mucines de sécrétion Julenius, {2005 #297}. De même, le mode de fixation des groupe H sur les cellules épithéliales chez le porc (36) ou dans les sécrétions sous forme de mucine (46) (47) a été rapportée.

Une des approches consiste à utiliser des traitements visant à libérer le corps H de son support. Une autre approche consiste à retirer les motifs protidiques ou lipidiques sur lesquels les motifs sont fixés. Il est possible de faire disparaître le core H en utilisant une enzyme comme la neuraminidase (47 ou une enzyme type alpha-L-fucosidase. Une autre méthode chimique consiste à faire disparaître certains résidus sucrés du système ABH du tissu comme la méthode décrite par {Derevitskaya, 1983 #294) pour fragmenter le core H du porc. Utilisation de méthode chimique pour faire détacher le core H par une beta élimination de la chaine sucrée suivie d'une bromination du groupe enamine résultant du clivage du groupe brominated amino acid residu par alkaline sodium borohydride technique utilisée pour la fragmentation du core H du porc (55). Possible utilisation d'un traitement par methanol-ethyl ether (1 :1, v/v) and chloroform-methanol (1 :1, v/v) (*cf K.Kishi and* S. *Iseki Immunochemical studies on blood group H and B substances from human hair*). L'ensemble de ces procédés peuvent être employés sur les bioprothèses.

### Utilisation d'animaux génétiquement modifiés pour le système ABH porcin ou exprimant des antigènes du système ABO humain:

Afin d'améliorer les compatibilités on pourra avoir recours à des porcs génétiquement modifiés, notamment pour les gènes ABH, pour les rendre compatible avec le système ABO humain.

On pourra par exemple sélectionner des porcs n'exprimant pas certains antigènes du groupe sanguin exprimés classiquement chez le porc (par exemple les antigènes du groupe A-L) ou faire des porc modifiés génétiquement pour ne pas exprimer tel antigène de groupe sanguin ou dérivés sucrée ou variable reliée à cet antigène ou de sur-exprimer tel autre antigène. Cela peut être également une régulation au niveau de l'expression d'un antigène du groupe sanguin avec sur ou sous expression. Il faut voir qu'il existe bien souvent une compétition entre différentes enzymes pour la réalisation de groupe sanguin au niveau de leur substrat. Ainsi le fait de générer en grande quantité tel ou tel résidu sucré fait qu'un autre antigène sucré ne sera peut-être pas exprimé. Production de porc n'exprimant pas par exemple d'antigènes comme l'antigène I par exemple.
Les animaux notamment les porcs peuvent être des animaux non génétiquement modifiés. Dans ce cas on étudiera les phénotypes des porcs et l'on sélectionnera les porcs d'intérêt en préférant si possible les races ou l'incidence de l'antigène phénotype désiré est élevée ou en sélectionnant pour une espèce donnée des individus intéressants ou au phénotype muté par exemple ou qui n'exprimerait par certains antigène ABH du phénotype porcin classique.

On peut également améliorer la compatibilité des porcs en utilisant des porcs transgéniques pour le système ABO humain en leur faisant exprimer notamment l'activité spécifique d'un gène du système ABO comme l'activité par exemple B transférase humaine pour faire un antigène B notamment chez des porcs de type H. On pourra également faire des porcs transgéniques pour augmenter l'expression des antigènes H, A ou B notamment dans les tissus désirés en les modifiant génétiquement pour exprimer le gène A transférase, B transférase ou « fucozyl transférase » (gène FUT) humain. On peut également faire des porcs KO pour des certains gènes du système ABH porcin (cf. antigènes de A à L entre autre).

Cette modification de l'antigénicité ABH peut être également associée à une modification d'antigénicité vis-à-vis d'autres motifs antigéniques comme des résidus sucrés alphaGal(Galα1,3Galβ1,4GlcNAcβ1-R) (« αGal »). Cela peut etre obtenu avec des porcs génétiquement modifiés comme des porcs KO pour la transmission de l'alpha Galactosyltransférase, ou traiter les tissus avec des enzymes comme l'alpha Galactosyltranférase, entre autres. On pourra également avoir recours à d'autres porcs modifiés vis-à-vis de résidus sucrés ou d'antigènes impliqués dans le rejet comme des porcs n'exprimant pas l'antigène majeur du rejet hyper aigu vasculaire xénogénique : alphaGal(Galα1,3Galβ1,4GlcNAcβ1-R) (« αGal »).

Ces modifications peuvent être associées ou couplées à d'autres méthodes visant à modifier l'expression d'autres résidus sucrés ou l'expression d'antigènes à la surface des bioprothèses appartenant ou pas au système ABH. Cette méthode peut être couplée à d'autres approches visant à modifier l'immunogénicité, la calcification, les propriétés physiques, la toxicité, la biocompatibilité, la trombogénicité des tissus implantés. Egalement des techniques visant à améliorer la qualité de la fixation incluant sa stabilité dans le temps.

Les différentes approches de traitements peuvent être combinées pour avoir un tissu ABH porcin/ABO-ABH humain de phénotype déterminé.

### 2) Fixation/Réticulation du tissu au phénotype ABH animaux/ABO-ABH humain déterminé :

Le tissu animal ABH compatible est prélevé de façon stérile et préparé pour fixation et traitement. Dans l'invention suivante nous proposons d'utiliser la classique fixation par le glutaraldehyde mais de nombreuses modifications de cette fixation ou l'utilisation, d'autres agents de fixations classiquement utilisés pour réaliser des bioprothèses peuvent être employés.

Le tissu est lavé avec de l'eau stérile ou en PBS. Le tissu peut être traité avec des surfactant avant la fixation pour retirer les lipides, les acides gras, cholestérol etc. (cf. US Patent 4553974). Fixation du tissu dans une solution de glutaraldehyde 0.625% pH 7.4 dans tampon phosphate pour 2 semaines pression de fixation inférieure à 2 mmHg contrôle de la température à 37°C. Le tissu est ensuite traité par un mélange de dénaturant/surfactant/crosslinking pour 3 jours (cf. respectivement Formaldehyde 4%, Ethanol 2.2% Tween 80 1.2%).

La fixation de tissu porcin par un traitement par glutaraldehyde puis traitement par Formaldehyde/Etanol/tween a été proposé depuis 1984 (6). Différentes fixations avec le glutaraldehyde ont été proposées depuis, ainsi que différents agents dénaturants (par exemple US patent 20070255423). Des agents anti-calcifiants sont souvent associés. Des prétraitements chimique par aluminium chloride et/ou ethanol sont parfois utilisés. Des surfactant peuvent être utilisé (cf. comme par exemple Dodécyl sulfate, acide alphaoleïque, acide homocystéique / US Patent 20060154230). Le tissu peut alors être stérilisé (par exemple US patent 743850, 6203755) et des solutions de préservation peuvent être utilisées (par exemple US patent 5935168; 20040136965, 7129035, 7014655, 6861211, 7579381, 7214344, 6878168, 6561970, 6547827, 6214054, 6008292, 5935168, 5931969, 5782931, 5215541, 4885005, 4838888, 4648881, 4647283). Bien évidement d'autres modes de fixation ou des variantes de cette fixation peuvent être utilisés avec variation du pH de la fixation au glutaraldehyde, utilisation d'un réducteur, fixation à la chaleur, etc. (Par exemple US Patents 7579381, 7214344, 6878168, 6561970, 6547827, 6214054, 6008292, 5935168, 5931969, 5782931, 5215521, 4885005, 4838888, 4648881, 4647283, 20070255423, 20060217805, 20040253291, 20070255423, 20050071926, 7214344, 20090164005).

*Autres agents réticulants*: cette polymérisation ou réticulation peut provenir d'une réaction chimique avec des agents réticulants connus et des dérivés et/ou leurs analogues, dérivés et combinaison comme par exemple génipine, aglycone d'acide nordihydroguaiarétique, acide geniposidique, composés époxydes, amidon dialdéhyde, glutaraldéhyde, formaldéhyde, subérimidate diméthylique, carbodiimides, acyl azide, « dye mediated photooxydation », succinimidyles, diisocyanates, azoture d'acyle, glycéraldéhyde, cyanamide, diimides, adipimidate diméthylique, rutérine, acide nordihydroguaiarétique, transformation enzymatique, thrombine, traitement dehydrothermique, réticulation endogène par des cellules et leurs produits biochimiques normaux (tels que la lysine oxydase produite par une cellule ...), méthodes visant à éliminer le glutarldehyde non fixé ou à diminuer la libération de résidus glutaraldehyde en bloquant avec un composant amine comme par exemple l'emploi de diphosphonates fixés sur tissu préalablement fixés au glutaraldehyde ou administrés directement dans le tissu traité, acide substitué avec groupement aliphatique « amino-substituted aliphatic functional acid» fixé de manière covalente au tissu fixé dans le glutaraldehyde, sels riches en fer ou stannique « ferric or stannic salts » avant ou après fixation au glutaraldehyde, traitement du tissu avec des sulfates polysaccharidiques "sulfated polysaccharides » comme par exemple chondroïtine sulfate, utilisation d'un traitement réticulant chitosan/heparine avant ou après fixation par glutaraldehyde, sels ou polymères « surtout elastomeric polymers», utilisation de solutions riches en phosphate ester or sels d'amonium quaternaire ou riches en alcool aliphatique sulfatés "sulfated higher aliphatic alcohol » après fixation au glutaraldehyde ou association de certains de ces procédés. Différentes réticulations ont été proposées : (par exemple US Patent 7579381, 20050071926, 6214054, 7214344,20090164005). Exemple d'autres agents fixateurs ou réticulant: Sulfo-NHS (par exemple US patent 7479164, 5733339), Triglycidylamine (TGA) (cf. US patent 20030196274, 7156881), Bis-maleimide (cf. US patent 6596471), Génipine (par exemple US patent 20020091445, 6998418, 6545042), epoxide (par exemple US patent 7014655, .6106555, 5080670), autre fixations (par exemple US patent 5094661, 5002566, 4976733, 5679112, 5447536, 5368608, 6322593, 6302909, 66231614, 6193749, 6177514, 6156531, 6132986, 6093530, 5919472, 20060207031, 200500719326, 2003010746, 6471723).

*Autres traitements associés* : des tissus dé-cellularisés peuvent être utilisés (par exemple US patent 20040052830, 20030228692, 5632778, 5613982. En plus des agents réticulant ou crosslinkant d'autres agents chimiques ou physiques, biologiques comme enzymatiques peuvent être associés comme des agents réducteurs, des agents types détergents, des agents visant à dé-cellulariser le tissu initial, des agents visant à retirer les lipides (par exemple US patent 6350732, 20040253291), à améliorer la fixation au glutaraldehyde en la rendant irréversible (par exemple US patent 6479079).

Les différents modes de réticulations, traitements ou procédés peuvent être associés.

### 3) Fabrication du dispositif bioprothétique à partir du tissus animal au phénotype ABH animal/ABH-ABO humain déterminé et fixé/réticulé :

Dans la présente invention nous proposons la réalisation d'un dispositif implantable comme une bioprothèse valvulaire avec le tissu précédemment obtenu. Le tissu avec un ABH déterminé est alors transporté dans une chambre stérile et travaillé, affiné ou configuré et attaché ou assemblé à n'importe quel composant biologique ou non biologique (par exemple stent, support, montant, anneau, conduit, segment de mesh en polyester etc.... ) pour former un dispositif bioprothétique. La bioprothèse se compose de trois valves, d'une valvule aortique de porc fixées sur une armature métallique circulaire en alliage de cobalt et de chrome disposant de trois montants reproduisant les commissures de l'anneau aortique. Ce stent est flexible afin d'amortir partiellement le stress imposé à la valve notamment au niveau des commissures. Les trois valves sont découpées du culot aortique, débarrassées au maximum des structures avoisinantes, et le reste du muscle directement incorporé dans la paroi du stent. La collerette est en silicone et est recouverte comme tout le stent de PTFE tressé. Dans d'autres cas, la bioprothèse n'a pas besoin d'être reconstituée car tout le culot aortique de porc va être utilisé en monobloc (en incluant les sigmoïdes aortiques, l'anneau et les sinus aortique).

D'autres bioprothèses peuvent être réalisées à partir du tissu préalablement obtenu. Exemples non exclusif de ces dispositifs sont: *bioprothèses avec stent* : La valve de Carpentier-Edwards porcine (Edwards lifescience) commercialisée depuis 1975 initialement fixée avec du glutaraldehyde, puis depuis 1984 fixation par glutaraldehyde et un traitement par Formaldehyde, Ethanol, Tween. Les valves Carpentier-Edwards RTM stented porcine bioprosthesis, bioprothèse en péricarde de boeuf (cf. Carpentier-Edwards .RTM pericardial Bioprosthesis™), valve de Hancock (Medtronic™) bioprothèse porcine fixée avec du glutaraldehyde 0.625% (Hancock I™) ou Glutaraldehyde et surfactant dodécyl sulfate (Hancock II™), valve Mosaïc™ (Medtronic ™) bioprothèse porcine glutaraldehyde avec acide alphaoléique fixation basse pression, valves Biocor™ et Epie™ (Saint Jude™), valve mitroflow ™ (carbomedix™) , valve pericarbon ™ (Sorin) pericarde de veau fixé au glutaraldehyde et post traitement à base de acide homocystéinique, modèle supra annumaire Soprano™, *stenless porcine aortique prosthèses* (cf. Edwards .RTM), valve free style ™ (Medtronic™) racine aortique de porc fixé au glutaraldehyde avec un agent anticalcique type alphaoleïque, Valve Toronto™ (Saint Jude Medical™) valve aortique de porc fixée dans du glutaraldehyde, valve Prima™ (Edwards™) racine aortique de porc fixée dans glutaraldehyde basse pression, valve Pericarbon Freedoom™ (Sorin) valve constituée par l'assemblage de deux feuillets de péricarde de veau et fixation glutaraldehyde et postfixation acide homocystéique, Valve CryoLife -O'Brien™ formée de trois sinus non coronaire porcins, ATS 3f™, *bioprothèse mise en place par voie endovasculaire* Core valve ™ (CoreValve, Inc, Paris, France) (WO03079929),.

*Principales bioprothèses cardiaques actuelles:* « Surgical Technology International » www. Surgicaitechnology.com STI XV Cardiovascular surgery « Advanced Technologies for cardiac valve replacement, transcatheter innovation and reconstructive surgery » W.R. Jamieson E, Hancock standard and Carpentier-Edwards standard, Carpentier-Edwards Supra-Annular (SAV) Aortic Porcine Bioprosthesis,The Carpentier-Edwards PERIMOUNT pericardial bioprosthesis (Edwards Lifesciences, Irvine, CA, USA), Carpentier-Edwards Duraflex Low-Pressure Mitral Bioprosthesis,Carpentier-Edwards PERIMOUNT Magna Aortic & Mitral Bioprosthesis, Carpentier-Edwards PERIMOUNT Plus Mitral Pericardial Bioprosthesis, Carpentier-Edwards PERIMOUNT Theon Mitral Replacement System, Edwards Prima™ Plus Stentless Porcine Bioprosthesis,Carpentier-Edwards Biophysio Pericardial Aortic Bioprosthesis, The Hancock II porcine bioprosthesis (Medtronic, Inc., Minneapolis, MN, USA), The (Medtronic Mosaic™ porcine bioprosthesis (Medtronic, Inc., Minneapolis, MN, USA), The Medtronic Mosaic Ultra™ (Medtronic, Inc., Minneapolis, MN, USA), (Medtronic Freestyle™ Stentless Porcine Bioprosthesis,The Medtronic-Venpro Contegra™ pulmonary valved conduit (Medtronic, Inc., Minneapolis, MN, USA), he St. Jude Medical-Biocor porcine bioprosthesis (St. Jude Medical, Inc., Belo Horizonte, MG, Brazil), St. Jude Medical-Biocor Supra Porcine Bioprosthesis,The St. Jude Medical Epic porcine bioprosthesis (St. Jude Medical, Inc., St. Paul, MN, USA,St. Jude Medical Epic Supra Porcine Bioprosthesis, The St. Jude Medical-Toronto SPV stentless porcine bioprosthesis (St. Jude Médical, Inc., St. Paul, MN, USA), the St. Jude Medical-Toronto Stentless Root™ porcine bioprosthesis (St. Jude Medical, Inc., Minneapolis, MN, USA), the' St. Jude Medical-Biocor pericardial bioprosthesis (St. Jude Medical, Inc., Belo Horizonte, MG, Brazil), the St. Jude Medical-Biocor stentless porcine bioprosthesis (St. Jude Médical, Belo Horizonte, MG, Brazil), the St. Jude Medical Trifecta™ pericardial bioprosthesis (St. Jude Medical, Inc., St. Paul, MN, USA), the Sorin Pericarbon™ MØRE pericardial bioprosthesis (Sorin Biomedica, Saluggia, Italy), the Sorin Pericarbon™ Freedom stentless pericardial bioprosthesis (Sorin Biomedica, Saluggia, Italy), the Sorin Pericarbon™ Freedom Solo stentless pericardial bioprosthesis (Sorin Biomedica, Saluggia, Italy), the Soprano supra-annular aortic pericardial bioprosthesis (Sorin Biomedica, Saluggia, Italy), the Mitroflow pericardial bioprosthesis (Sorin-Mitroflow, Richmond, British Columbia, Canada), the CryoValve Aortic valve with or without conduit (Cryolife International, Inc., Kennesaw, GA, USA), the CryoValve Mitral valve (Cryolife International, Inc., Kennesaw, GA, USA), the Cryolife-O'Brien stentless porcine bioprosthesis (Cryolife, Inc., Kennesaw, GA, USA), the Shelhigh Skeletonized Super-Stentless™ (Shelhigh, Inc., Union, NJ, USA), the BioMitral™ valve (Model NR-900), the current generation Shelhigh porcine pulmonic valve conduit (Shelhigh, Inc., Union, NJ, USA), the Koehler Aspire porcine bioprosthesis (Koehler, Bellshill, Scotland), the Koehler Elan stentless aortic porcine bioprosthesis (Koehler, Bellshill, Scotland), the Koehler Root Elan stentless aortic porcine bioprosthesis (Koehler, Bellshill, Scotland), the 3F Therapeutics™ bioprosthesis (3F Therapeutics Inc., Lake Forest, CA, USA), the Labcor stented porcine bioprosthesis (Labcor, Inc., Belo Horizonte, MG, Brazil), the Labcor stented pericardial bioprosthesis (Labcor, Inc., Belo Horizonte, MG, Brazil), the Labcor stentless porcine bioprosthesis (Labcor, Inc., Belo Horizonte, MG, Brazil), the Glycar Quattro™ mitral bioprosthesis (Glycar, Inc., Johannesburg, South Africa).

*Principaux brevets correspondant à* ces *inventions* de façon non exclusive : différents modes pour la réalisation de bioprothèses aortiques (par exemple US patent 7316712, 6391538, 20020173843, 5824061, 20090030511, 6254636, 6086612, 6719789, 6074417, 7011681 , 6530952, 5824067, 20040024452, 5769780, 4692164, 4626255, 20080154358, 5824060, 7166124, 7163556, 6540781, 5728152, 5571174, 5549665, 5352240) , mitrale, pulmonaire 7320705, tricuspide, avec ou sans stent (par exemple US patent 5156621, 5080670, 4626255, 4561129, 4388735, 4378224), avec des stents résorbables (par exemple US patent 5489297 avec ou sans suture (par exemple 20030196274, 20030181974, 7322932, 6027530, associée ou non à des stents (par exemple 20090118826) pour des implantations endovasculaires (par exemple 20030125805, 20030125793, 7125418, 7318998, 7041132, 7033390, 6719785, 6682558, 6087552, 5755782, 554521) ou par chirurgie mini-invasive ont été rapportées. Egalement des façons pour monter les feuillets pour former les valves. Possibilité de fabriquer des valves. Des systèmes de support (par exemple US Patent 20010002445). Possibilité d'utiliser des stimulations physiques lors de la fabrication des bioprothèses (par exemple US Patent 7348175). Système de réparation valvulaire (par exemple US patent 20040143323, 7455689) ou pour fabriquer in situ la bioprothèse (par exemple US patent 5326370)(The Edwards Lifesciences percutaneous aortic heart valve (Edwards Lifesciences, Irvine, CA, USA) The CoreValve percutaneous pericardial aortic valve (CoreValve, Irvine, CA, USA) Medtronic Melody transcatheter pulmonary valve (Medtronic Inc., Minneapolis, MN, USA) The ENABLE™ Aortic Bioprosthesis (3F Therapeutics, Lake Forest, CA, USA) The ENTRATA™ transventricular aortic bioprosthesis (3F Therapeutics, Lake Forest, CA, USA) The Edwards Ascendra™ valve replacement system (Edwards Lifesciences, Irvine, CA, USA) The Sadra Percutaneous pericardial aortic valve (Sadra Medical, Campbell, CA, USA) i The AorTx (AorTx, Palo Alto, CA, USA) concept The Corazôn PAVR system (Corazón Technologies, Menlo Park, CA, USA) The Corazón Surgical Aortic Valve Repair (SAVR) System (Corazón Technologies, Menlo Park, CA, USA) Percutaneous bioprostheses for tricuspid regurgitation (3F Therapeutics, Lake Forest, CA, USA)).

*Exemple d'autres dispositifs bioprothétiques_*: (liste non exhaustive) : possibilité de réaliser des matrices patch (par exemple US Patent 20010051824, 20040157206, 6652583, 6174333, 5855620, 6517576), Cook Biotech Inc, West Lafayette, IN, USA, SMJ™ Pericardial Patch with EnCap™ Technology St Jude Medical™), des conduits vasculaires (par exemple US patent 5545215, 20040158320, 20010020191, 6358275, 6206917, 6110212, 6087552), conduits valvés (par exemple US patent 5376112) ou pour traiter des tissus biologiques (par exemple US patent 20060207031).

La bioprothèse peut être un échafaudage, comme par exemple un échafaudage décrit dans PCT/FR2008000785. Cet échafaudage peut être cellularisé; Il peut être utilisé pour la thérapie cellulaire, la régénération, remplacement reconstruction tissulaire y compris cutané. L'échafaudage tridimensionnel est totalement ou partiellement ou non-biodégradable. L'échafaudage tridimensionnel est formé dans une phase liquide qui, une fois délivrée, après ou sans activation peut se transformer en phase solide (par exemple solution, pâte, gel, colloïde en suspension, plasma). L'échafaudage tridimensionnel peut être fait d'un hydrogel constitué d'acides aminés hydrophobes et hydrophiles capables de s'assembler spontanément en structures macroscopiques. L'échafaudage tridimensionnel peut être un gel ou un agent tensioactif. L'échafaudage tridimensionnel où l'échafaudage est un agent tensioactif ou un « agent intelligent », c'est à dire une matière biologique constituée de structures assemblées spontanément à grande échelle reposant sur des interactions locales au niveau moléculaire. Dans l'échafaudage tridimensionnel, la construction 3D peut être obtenue par superposition de cultures obtenues sur des échafaudages 2D différents. L'adhérence des cellules à ce support 2D peut être modulable. Ces supports 2D peuvent contenir des collagène/fibrine/fibrinogène modifiés par fixation de molécules d'adhésion. Plusieurs échafaudages 3D de natures différentes peuvent également être superposés de manière séquentielle ou pas. L'échafaudage tridimensionnel peut former une matrice de cellules où la construction de tissu artificiel contient des biomatériaux de formes choisies facilitant le regroupement structurel : micro ou nano structures (par exemple les tubes micro ou nano, les nanoparticules, les micros ou nanopores. Les microparticules ou les nanoparticules sont faites du silicium, poly -(acide lactique) mélange d'acide Co polymère - acide glycolique lactique, cyclodextrine, liposome conjugué ou pas aux nanoparticules quantum dot, magnétite, filaments, des analogues structuraux pour former l'interface extérieure, analogues peptidiques structures β-/ou-α qui forment des filaments ou tubes, d'éponge, de poudre, de conduit, de sphère, de microsphère, de film, micro ou nanofibrilles, membrane de lipide, fibre, maille, matrice, patch, feuille de tissu, ouatine ou combinaison.

Autres dispositifs bioprothétiques comme (par exemple US patent 5067962, 6936070, 6790213, 4585458, 7404819), The Shelhigh BioRing (Shelhigh, Inc., Milburn, NJ, USA) Prima™, Restore™, Oasis™, Surgisis™, CuffPatch™, GraftJacket™, Alloderm™, TissueMend™, OrthAdapt™. Le dispositif peut être également du collagène injectable ou pas (par exemple US patent 6548077, 6127143, 5814328, 5374539), tissu compressé par exemple (par exemple US patent 7141064). Il peut servir à des régénérations, remplacement, reconstruction tissulaire comme par exemple de la chirurgie de reconstruction avec des bioprothèses fabriquées à partir de tissus cutanés de porc.

Les différents dispositifs décrits ci-dessus peuvent être associés.

### Principes régissant les règles de compatibilité phénotypique dans le système ABO/ABH

Il s'agit donc pour un patient de « phénotype ABO » donné d'implanter un tissu ou extrait tissulaire susceptible d'induire chez ce patient le moins de réactivité possible en faisant en sorte que le phénotype ABH du dispositif bioprothétique soit « compatible » avec le système ABO du patient.

Le phénotype est celui exprimé au niveau du dispositif bioprothétique au moment de son implantation et qui peut être différents du phénotype ABH de l'animal ou des animaux chez lesquels les tissus ont été préparés. En fonction ou pas de la présence de l'antigène A, B les individus ont ou pas dans leur sang une réactivité immunologique vis de l'antigène manquant. Cette réactivité s'exprime par la présence dans le sang de ces individus d'anticorps vis-à-vis de l'antigène maquant. Mis à part les individus du « groupe Bombay » qui n'ont pas d'antigène H et donc pas de groupe A et B et ont donc des anticorps anti H anti A et anti B, tous les être humains expriment la spécificité H. Ils n'ont donc pas d'anticorps anti H. Les patients du groupe A ont donc une réactivité anti B mais acceptent les tissus H ou A. Les patients du groupe B ont une réactivité anti A mais acceptent les tissus B et H. Les patients du groupe O ont une réactivité anti A et anti B et acceptent les tissus de type H. Les patients du groupe AB n'ont pas de réactivité anti A, anti B ou anti H et acceptent donc les tissus A, B ou H. Les patients du « groupe Bombay » ont une réactivité anti A, anti B et anti H. La réactivité est vis-à-vis de l'antigène Humain A, B, H. S'il existe une identité pour l'antigène A et dans une certaine mesure avec l'antigène H chez l'homme et certains animaux comme le porc par exemple, cela n'est pas vrai pour l'antigène B qui est différent. Les individus du groupe B humain vont donc développer une réaction immunologique vis-à-vis de tissu du groupe B animal. Pour les autres groupes sanguins exprimés chez le porc notamment le groupe I qui est fréquemment présent chez les porcs n'exprimant pas l'antigénicité A, les individus vont développer une réactivité vis-à-vis de cet antigène.
Les différentes antigénicités ont été définies précédemment.

La présente invention est en outre illustrée, sans y être limitée, aux exemples suivants.

### EXEMPLES

### Exemple 1: Obtention d'un tissu animal avec un phénotype ABH animaux/ABO-ABH humain déterminé

Dans l'exemple 1, nous avons utilisé la propriété de certains porcs d'exprimer l'antigénicité A au niveau de leur tissu pour obtenir des tissus avec un « phénotype A humain » mais d'autres approches pour obtenir des tissus avec une spécificité donnée ont été décrites précédemment dans le brevet.

Dans l'exemple 1, nous avons utilisé des PCR sur de l'ADN extrait de valvule de porc pour détecter l'activité A transférase humaine qui code pour la spécificité A en utilisant le kit d'extraction de l'ADN de Invitrogen™ et l'ADN a été amplifié en utilisant 3 paires de « primers » (amorces) connues pour amplifier le gènes A1 humain (56). Mais d'autres primers ou amorces peuvent être utilisées comme FY-520 (5'-CCGGAATTCAACACTTCATGGTGGGACAC-3' - SEQ ID N° 1) et FY-521 (5'-CCGGAATTCTA GCTCTCATCATGCCACAC-3' - SEQ ID N°2). D'autres approches comme des approches histologiques peuvent être utilisée pour phénotyper les porcs.

### A. Méthode pour obtenir le phénotype ABH d'un tissu animal :

La méthode d'identification des groupes ABH chez les animaux est relativement commune et beaucoup de techniques appliquées au phénotype ABO chez l'homme sont applicables chez l'animal. Les techniques utilisées chez le porc peuvent être étendues à d'autres espèces notamment le boeuf. En effet le système ABO /ABH est un système qui a été très préservé au cours de l'évolution.

### B. Détection du groupe sanguin du porc :

Pour déterminer le groupe sanguin du porc. Prendre sur tube hépariné 0.5 ml de sang de porc et 0.5 ml de PBS. Rajouter 25 microlitre de sang dilué et le mettre dans un tube Eppendorf pour centrifugation. 25 microlitre d'anticorps de souris anti humain A est incubée, incubé à température ambiante pour 5 min., puis centrifugé à 900g pour 3 min. Après centrifugation, l'hemagglutination est observé sous un microscope conventionnel. L'incubation avec des anti corps anti B humain sert de contrôle négatif (51).

Il existe en parallèle chez le porc comme chez de nombreux animaux un système ABH tissulaire avec une certaine corrélation entre le système ABH sanguin et tissulaire (43).

Certains tissus sont plus prompts que d'autres à exprimer les antigènes ABH. Les tissus exocrines, les épithéliums du tube digestif, l'appareil urinaire et notamment les reins, l'appareil génital y compris testicule, les glande salivaires.

### C. Détection du groupe AH du porc sur des tissus fixés :

Des biopsies de reins ont été congelées en O.C.T. milieu de cryocongélation (Miles, Inc., Elkhart, IN, USA) dans l'azote liquide. Les prélèvements ont été coupés puis préservés à -80°C. Les sections ont été secondairement fixées avec de l'acétone froid pour 10 min. desséchées à l'air ambiant puis réhydratées en PBS pour 7 min.. Pour limiter les bruits de fond les lames ont été incubées pour des périodes de 15 minutes avec les différents agents 3% hydrogène peroxyde (Sigma) and avidin (Dakocytoformation) protéine blocking agents (Thermo, Electron, Pittsburgh PA, USA). Entre chaque bloque, rinçage pendant 10 min.. Les lames ont alors été incubées à température ambiante pour 30 min. avec un anticorps primaire anti A ou anti H (DakoCytoformation). Après lavage, les sections ont été secondairement incubées avec un anticorps secondaire avec un anticorps de mouton anti souris « biotinylated » pour 30 min (DakoCytoformation). La réaction colorée a été réalisée en utilisant le 3-amino-9-ethylcarbazole qui va réagir avec la peroxydase pour donner une coloration rouge et les lames ont également été marquées avec H∼E (hématoxyline Eosine) (51).

Autre solution utilisant une fixation au paraformaldehyde puis un traitement à la microonde puis utilisation kit de détection non biotynilé (Vision detection kit HRP/DAB (Dako, H5007)) (58).

La détection de l'antigène H peut se faire en utilisant une lectine spécifique Ulex Europaeusl (UEAI)(Vector Laboratories, Burlingame, CA, USA) ou des anticorps anti-H anti-H1 ou anti-H2 (cf. Oriol Transplant international 1994). L'antigène porcin I peut également être identifié en utilisant des lectines comme la lectine Galactose-specific rictin lectin (RCA₁₂₀) (52), l'Arachis Hypogaea, Helix pomatia (HPA San Mateo CA USA) et anticorps pour le groupe A. Globalement dans la race large white pigs (analyse sur 37 porcs) 51% des porcs sont du groupe A (Marquage) anticorps. 38% ne sont pas reconnus par l'anti A mais par contre exprime la chaîne H surtout de type 2 reconnue par la lectine UEAI. Enfin 11% des porcs sont A- et H- mais sont reconnus par une autre lectine (cf. Arachis Hypogaea (PNA) et anticorps anti-I+), sont généralement le^{y-}/le^{x+}..

Il existe cependant même chez les porcs de type A+ un certains nombre qui sont également I+. Les porcs de type A sont généralement H-car ils ne réagissent pas avec UEA 15/19. Il existe cependant 3/19 de ces porcs qui expriment l'antigène H et A humain. Ils sont A+/H+/I-. Il existe également parmi ces Porc de phénotype A 6/19 ∼30% de porcs qui ne sont pas de pur groupe A et qui expriment également le groupe I spécifique du porc. Ces porcs sont A+/H- /I+ (49).

Généralement les porcs qui sont H+ expriment également la spécificité. Lewis et sont généralement le^{x-}/ley⁺. Pour le groupe A-H+ 3/14 sont également I+ (49). Dans une autre analyse Busch J. sur des biopsies de rein de porc (Large White/Landrace/Duroc) cross-breed pigs montre que A+ 8/11 avec 2/8 (25%) A+ H+. Les autres 3/11 sont A-H+ (51).

Pour déterminer le type de core H1 ou H2 de la chaîne H, la résistance des porcs à différentes toxines « heat lable toxine from E. Coli » (LTs) ou Choiera (CT) par exemple pourrait être utilisée (on pourra aussi utiliser des anticorps spécifiques anti-H1 ou anti-H2 soit sur les tissus de ces animaux soit sur des protéines secretées par ces animaux). La capacité de protéines purifiées à partir de secrétions présentent à la surface de l'intestin de porc à fixer ces toxines serait directement liée au phénotype H1 ou H2 du core H du porc considéré. En l'absence de fixation, il s'agit de porc I. Fixation de LTs uniquement core H2. Fixation de LTs et de CT il s'agit de core H1. Ce type d'approche peut être une manière rapide d'identifier les porcs pour leur phénotype H du fait d'une plus ou moins grande résistance à l'infection (52, 59, 60) ou par certains comportement comme par exemple la taille de la litière (61). Les E. Coli possèdent un récepteur qui reconnaît spécifiquement les cores A H1. Cette propriété pourrait être utilisée également pour identifier les porcs de core H1 (62).

On pourra aussi s'intéresser à la spécificité Lewis de ces animaux surtout sur biopsie des testicules chez des animaux âgés de plus de 3 semaines (possibilité d'utiliser notamment les anticorps anti. le^{x} ou anti le^{y}). La spécificité lewis est codée par le gène FUT2 qui code également pour le core type H2 de la chaîne H. Les glandes gustatives permettent également de détecter la majorité du panel des antigènes ABO (63).

Les tissus sont fixés en formaline 10% et inclus en paraffine pour sections. Après deparaffinisation, les sections ont été incubées pendant 30 min. en chambre humide avec une lectine conjuguées fluorescein or rhodamine 20 mg /ml puis monté en milieu pour fluorescence (Vector Laboratories, Burlingame , CA, USA) et examiné sous microscope à fluorescence (43, 49).

La détection peut se faire dans sur les biopsies de nombreux tissus notamment les tissus exocrines, appareil génito-urinaire(51), tube digestifs (49), appareil digestif. Pour revue Nishi K. et al. 2005 ABO Blood Typing (64).

### D. Détection du phénotype ABH sur les produits de secrétion de l'animal

Il est possible également de faire une analyse des substances H sur les secrétions elle-mêmes comme par exemple le lait (65), les secrétions sous maxillaire, le mucus bronchiques. Les groupes ABO sont souvent associés à des mucines. Possibilité d'identifier les différentes formes par HPLC (36) ou western Blott avec utilisation d'anticorps spécifique anti H1 (Clone 17-206 Signet Dedham MA, USA) ou anti H2 clone 92 FR-A2 Dako Carpinteria, CA, USA) ou Le^{b} (Clone T218 Signet Dedham MA, USA), Le^{a} (clone KM231 Calbiochem-Novabiochem San Diego CA), Le^{x} (Clone P12 Calbiochem-Novabiochem San Diego CA) (Clonex (65). Si la majorité des porcs sécrètent dans leur lait des cores H de type H1, la sécrétion du core H de type H2 est plus restreinte (65).

### E. Détection du groupe AH du porc sur les cellules épithéliales des muqueuses :

Une autre technique plus facile est de faire un phénotypage sur les cellules épithéliales de la muqueuse buccale en frottant la muqueuse buccale avec un coton et en appliquant ce coton sur une lame de verre. Les spécimens sont laissés à sécher à la température ambiante. Les lames sont fixées avec de l'acétone froid (Sigma, St Louis, MO, USA) pour 10 min. puis réhydratées en PBS. Un anticorps de souris anti humain A 1/50 Dilution, H 1/50 dilution IgM anticorps monoclonal (DakoCytoformation, Carpinteria, CA, USA) est appliqué 100 microlitre dans une chambre humidificatrice pour 60 min.. puis incubation pour 60 min. dans le noir avec un anticorps FITC conjugué mouton anti souris 1/100 (Vector, Burlingame, CA, USA) est observés avec un microscope à florescence (51).

### F. Détection des systèmes ABH sur les phanères :

L'antigène A a été montré comme exprimé dans l'intérieur de phanères comme les poils (64).

### G. Genotypage des animaux pour leur système ABH :

Du fait de la complexité des groupes ABO, Les techniques de biologie moléculaire (31-34) risquent, dans quelques années, de remplacer les technique actuelles par sérologie. Ces techniques peuvent être utilisées chez l'animal (66).

Il est aussi possible d'effectuer un phénotype du porc par genotyping (56, 57, 63, 67-69). L'ensemble de l'élaboration des ces sucres est sous la commande de différentes enzymes codés par des gènes. Dé manière très intéressante, les gènes codant pour ces différentes activités ont été très conservés au cours de l'évolution de telle sorte que dans beaucoup de cas les séquences d'amorces humaines que l'on utiliserait pour séquencer tel ou tel gène peuvent être utilisés chez l'animal. C'est notamment le cas pour l'endogalactosidase gène A (A1 ou A2) qui code pour l'antigène A humain (67). Pour la spécificité H il est possible de s'intéresser aux gènes FUT 1 et 2 notamment (42, 60, 70-72). Le « genotyping » pour les groupes ABH chez l'animal a été réalisé sur des sécrétions comme la salive (67), le sang, sur de l'ADN extrait de tissu comme du tissu sous maxillaire chez le porc (57). Le genotypage peut se rechercher sur le groupe ABO mais également le statut sécréteur non sécréteur (73).

Les différentes techniques précédemment décrites vont permettre d'identifier le ou les porcs intéressants du fait de leur spécificité ABH. Notamment les porcs A+ (A+H+I- > A+H-I- > A+H-I+ ), et les porcs H+ (A-H+I->A-H+I+) et les autres porcs notamment les porcs I (A-H-I+). Les résultats pourront être confirmés éventuellement sur coupe histologique de tissu. Cette approche pourra s'accompagner d'un phénotypage du core H éventuellement par génotype sur les glandes sous maxillaire par exemple.

Les bioprothèses seront alors par exemple proposées en fonction du phénotype type H1 ou H2 du core H du patient. Comme on l'a vu précédemment il existe une relation directe entre le type de core H, H1 ou H2 et le phénotype sécréteur ou pas du patient et ses caractéristiques pour le groupe Lewis Le^{a} ou Le^{b}.

### Exemple 2: Fixation/Réticulation du tissus au phénotype ABH anirnaux/ABO-ABH humain déterminé :

Les tissus valvulaires porcins précédemment prélevés chez des porc de type A+ ou A- dans l'exemple 1 ont été immergés dans une solution de glutaraldehyde 0.625% en PBS pH 7.4 avec une température contrôlée 37°C pour une durée de 2 semaines puis soumis à un traitement par agent dénaturant/surfactant et crosslinking (cf. respectivement formaldehyde 4%, Ethanol 2.2% and Tween 80 1.2%) pendant 3 jours.

Différentes possibilités de fixation de tissus ont été décrites précédemment et peuvent être utilisées à la place de la fixation que nous avons utilisé comme par exemple, de manière non exclusive, différents types de traitements pour fixer les tissus comme décrit précédemment. A titre d'exemple d'alternatives de fixation pour le glutaraldehyde (US Patent 7579381, 7214344, 6878168, 6561970, 6547827, 6214054, 6008292, 5935168, 5931969, 5782931, 5215521, 4885005, 4838888, 4648881, 4647283, 20070255423, 20060217805, 20040253291, 20070255423, 20050071926, 7214344, 20090164005).

### Exemple 3 : La connaissance du phénotype ABH du tissu réticulé permet d'obtenir des tissus bioprothétiques qui se calcifient moins chez le receveur en fonction de son groupe ABO/ABH:

Les rats comme les porcs expriment des antigénicités B ou AB. Les tissus porcins précédemment fixés de phénotype A + ou A- ont été implantés en sous cutané chez des rats Wistar de phénotype ABH A+ (cf. AB) ou A- (cf. B) de manière stérile.

Le phénotype des rats qui ont été implantés a été déterminé en faisant un marquage histologique sur biopsie des glandes sous mandibulaire: Les, tissus sous mandibulaires ont été fixés avec du formaldéhyde 10% mis en paraffine et des sections de 5 micromètres ont été réalisées. Des anticorps anti A (ortho diagnostic, Ranitan NJ) ont été utilisés comme précédemment décrit. (*cf*. J. Of Forenscic Medicine and toxicology 2001, 20, n°2 Nishimura A.).

La calcification des implants a été mesurée par absorption spectroscopiques après 30 jours d'implantation en sous cutané chez le rat. n=10 par groupe Calcifications implants A+ porcin chez les rats A- 135+/-22 µg Ca/mg tissue déshydraté. Calcium implants A+ porcin chez les rats A+ 30+/-7 µg Ca/mg tissue déshydraté.

Cet exemple démontre bien comment la connaissance du phénotype ABH du tissu d'origine permet d'obtenir des dispositifs qui vont avoir moins tendance à calcifier si l'on connaît le groupe ABO/ABH du receveur et si l'attribution se fait selon les critères que nous avons définis.

### Exemple 4 : Résultats de dispositifs bioprothétiques porcins en fonction du phénotype ABO des patients implantés :

### 4.1 Implication du phénotype ABO des patientes dans la longévité des bioprothèses :

Actuellement pour un individu donné, il n'existe que très peu de paramètres pour prédire la longévité d'une bioprothèses. Pour l'attribution d'une bioprothèse on tient compte du site d'implantation de l'âge, de la configuration du site d'implantation (gradient, encombrement) mais des paramètres propres au sujet comme sa réactivité immunologique ne sont pas pris en compte.

Dans l'étude suivante nous apportons la preuve que la connaissance du groupe ABO permet de déterminer la réactivité du sujet vis-à-vis de résidus sucrés ABH portés par le dispositif bioprothétique et que en prenant en compte cette réactivité et en adaptant les dispositifs, il est possible d'obtenir des dispositifs à la longévité supérieure avec moins d'échec précoce et moins de tendance à la calcification.

Des bioprothèses porcines type « Carpentier-Edwards standart » (traitement par glutaraldehyde ou traitement glutaraldehyde plus « traitement stérilisant (cf. formaldéhyde/Ethanol/Tween ») fabriquées à partir de porc de type A+ ou A- ont été implantés chez des patients de groupe A, O, B, AB.

Lors de l'implantation et lors de la réalisation de la bioprothèse, le phénotype du porc, n'était pas recherché car on ne savait pas comme on le démontre dans cette invention que le groupe ABH du porc pouvait avoir une influence ni que le groupe ABO du patient pouvait avoir une influence sur la longévité de la bioprothèse.

Etant donné la fréquence du phénotype A chez le porc environ 30% un patient du groupe A avait une chance sur 3 de recevoir une bioprothèse fabriquée chez un animal lui correspondant phénotypiquement. Cela passait à 1/6 avec des bioprothèse fabriquées à partir de deux porcs comme c'était le cas pour certaines bioprothèses commercialisée.

Nous avons donc investigué l'ensemble des bioprothèses explantées pour cause de dégénération sur un laps de temps de 10 années. Cela représente un nombre d'environ 920 patients. Chez ces patients les différents facteurs rapportés pour influer sur la longévité des bioprothèses (Age à l'implantation, sexe, diamètre de la bioprothèse, type de bioprothèse, site d'implantation (Aortique /mitral/ tricuspide), nombre de bioprothèses implantées ainsi que le groupe ABO dès patients a été recherché. Il y avait 52% d'hommes, site d'implantation 62% position aortique, 36% mitrale, 2% tricuspide. Nombre de bioprothèses implantées n=1 82%, n=2 17%, n=3 1%. Longévité de la bioprothèse <6ans (10.4%) ; [6-9] (27.3%) ; [9-12] (35.5%) ; [12-14] (13.9%); >14 (12.8%). La répartition des groupes ABO chez les patients porteurs de bioprothèses dégénératives 36.3% A, 42.4% groupe O, 15.2% groupe B, 6.1% groupe AB. Dans la population caucasienne d'où était issu ces patients la répartition des groupes ABO est respectivement de groupe A>40%, groupe O>40%, groupe B<10%, groupe AB ∼3%. Dans notre population de patients réopérés il y avait proportionnellement moins de patients de groupe A, le % des patients du groupe O est dans la normale et par contre les groupes B et AB se trouvent augmentés ce qui va dans le sens d'une plus incidence de ré-opérations chez les patients du groupe O, B ou AB par rapport aux patients du groupe A.

Nous avons alors réalisé une analyse multivariée sur l'ensemble des facteurs de risques classiquement rapportés comme ayant un impact sur la longévité de la bioprothèse jusqu'ici y en incluant en plus le groupe ABO des patients. Utilisation du logiciel SEM.

Nous avons retrouvé deux variables indépendantes pour expliquer la longévité des bioprothèses (cf. Tableau 1). le groupe sanguin du patient, le site d'implantation et le nombre de bioprothèses implantées. De manière fort intéressante, le facteur le plus important dans le model est le groupé ABO des patients.

**Tableau 1 : Analyse multivariée facteur de longévité de bioprothèse porcine : Implication groupe A par rapport aux autres groupes**

| **Longévité** | **Coef.** | **Std Err.** | **t** | **P(t)** | **Interval. 95%** |
|---|---|---|---|---|---|
| **Group A** | 0.79 | 0.33 | 2.33 | 0.02 | 1.45 |
| **Age implant**. | -0.01 | 0.01 | -0.94 | 0.34 | 0.01 |
| **Sexe** | 0.25 | 0.32 | 0.78 | 0.43 | 0.88 |
| **Site Mitral** | -0.76 | 0.33 | -2.30 | 0.02 | -0.11 |
| **Nombre bio.** | -0.44 | 0.35 | -1.25 | 0.21 | 0.25 |

Indépendamment de tous les autres facteurs les patients du groupe A ont une longévité de la bioprothèse 2.33 années supérieures aux patients des autres groupes. Cette différence est déjà importante puisqu'elle sort en premier dans l'analyse multivariée avec le poids le plus important. Elle est de la même importance que le site d'implantation de la bioprothèse (site d'implantation mitrale/aortique) qui est une donnée incontournable et reconnue par tout clinicien dans son choix pour l'implantation d'une bioprothèse.

La différence que nous rapportons sous-estime de façon considérable le bénéfice escompté. En effet selon les races des porcs d'origine, l'incidence du groupe A varie de 20-40%. De plus dans les groupes A il y a différents phénotypes A+-H+-I- A+-H+-I-, mais aussi A+-H-I+ -30%. Donc on peut estimer que la différence observée est due aux patients du groupe A qui ont reçu des tissus du porc soit seulement entre 14% et 30%. La différence réelle est probablement 3x supérieure par rapport aux chiffres que nous rapportons en termes de gain en années. Cette probabilité est encore 2 fois plus faible pour les patients du groupe A qui vont recevoir une bioprothèse préparée à partir de différents porcs.

Les patients du groupe O (tableau 2) n'ont pas spécialement une longévité prolongée de leur bioprothèses par rapport aux autres groupes. Il en est de même pour les groupes B (Tableau 3) et AB (Tableau 4). De manière intéressante, alors que le groupe B partage certaines similitudes structurales avec l'antigène majeur de xenorecativité : « alphaGal », les patients du groupe B n'ont pas une longévité spécialement plus marquée de leur bioprothèse.

**Tableau 2 : Analyse multivariée facteur de longévité de bioprothèse porcine : Implication groupe 0 par rapport aux autres groupes**

| **Longévité** | **Coef.** | **Std Err.** | **t** | **P(t)** | **Interval. 95%** |
|---|---|---|---|---|---|
| **Group 0** | 0.27 | 0.31 | 0.86 | 0.38 | 0.88 |
| **Age implant**. | -0.009 | 0.01 | -0.84 | 0.40 | 0.01 |
| **Sexe** | 0.17 | 0.32 | 0.54 | 0.59 | 0.80 |
| **Site Mitral** | -0.69 | 0.33 | -2.1 | 0.038 | -0.04 |
| **Nombre bio.** | -0.60 | 0.35 | -1.7 | 0.086 | 0.09 |

**Tableau 3 : Analyse multivariée facteur de longévité de bioprothèse porcine : Implication groupe B par rapport aux autres groupes**

| **Longévité** | **Coef.** | **Std Err.** | **t** | **P(t)** | **Interval. 95%** |
|---|---|---|---|---|---|
| **Group B** | 0.45 | 0.44 | 1.04 | 0.30 | 1.33 |
| **Age implant.** | -0.009 | 0.01 | -0.82 | 0.41 | 0.01 |
| Sexe | 0.18 | 0.32 | 0.57 | 0.57 | 0.82 |
| **Site Mitral** | -0.70 | 0.33 | -2.14 | 0.033 | -0.06 |
| **Nombre bio.** | -0.61 | 0.35 | 0.35 | 0.082 | 0.08 |

**Tableau 4 : Analyse multivariée facteur de longévité de bioprothèse porcine : Implication groupe AB par rapport aux autres groupes**

| **Longévité** | **Coef.** | **Std Err.** | **t** | **P(t)** | **Interval. 95%** |
|---|---|---|---|---|---|
| **Group AB** | **-0.38** | **0.68** | **-0.55** | **0.58** | **0.97** |
| **Age implant.** | **-0.009** | **0.01** | **-0.84** | **0.40** | **0.01** |
| **Sexe** | **0.18** | **0.32** | **0.58** | **0.56** | **0.81** |
| **Site Mitral** | **-0.72** | **0.33** | **-2.17** | **0.03** | **-0.06** |
| **Nombre bio.** | **-0.56** | **0.35** | **-1.6** | **0.11** | **0.13** |

Dans cette invention nous montrons l'implication des groupes ABO dans la longévité des bioprothèses. Nous montrons comment la prise en compte des groupes ABO des patients comme marqueurs de réactivité vis-à-vis de tissu hétérologue peu permettre d'obtenir des dispositifs médicaux/chirurgicaux comme des bioprothèses à longévité considérablement améliorée pour un patient donné. Le gain de longévité dépasse largement tous les résultats obtenus jusqu'alors avec les différents types de bioprothèses qui ont été développées et commercialisés.

### 4.2 Dans cette invention nous démontrons comment la connaissance du ABO des patients est un facteur clef pour déterminer les bioprothèses qui vont avoir des longévité considérées comme exceptionnelles ou des échecs précoces inexpliqués.

Dans cette invention nous montrons comment la prise en compte du groupe- ABO des patients permet d'obtenir chez un patient donné, en admettant qu'il reçoive le dispositif avec un phénotype ABH adapté, des dispositifs à durée prolongées et de limiter les échecs précoces dus à une altération précoce des bioprothèses avant 7 ans. Cela permet de réaliser des dispositifs plus sécurisés en termes de résultats, à la fois meilleurs sur le long terme, avec moins d'échecs précoces.

Nous avons analysé séparément le groupe de bioprothèses à longévité exceptionnelle au-delà de 16 ans en incluant l'ensemble des facteurs connus pour expliquer la longévité des bioprothèses et le groupe ABO des patients.

Encore une fois, comme le montre le tableau 5, le groupe ABO du patient est, en analyse multivariée, le principal facteur prédictif d'une longévité exceptionnelle d'une bioprothèse à plus de 16 ans. Ce qui est également très important c'est que là encore le groupe A apparaît comme le facteur prédictif le plus important de longévité des bioprothèses à long terme avant même le site d'implantation. Encore une fois, le type de bioprothèse implanté n'apparait pas comme une variable prédictive significative d'une longévité prolongée de la bioprothèse dans cette analyse.

**Tableau 5**

| **Longévité>16 ans** | **Coef.** | **Std Err.** | **T** | **P(t)** | **Interval. 95%** |
|---|---|---|---|---|---|
| **Group A** | 2.3 | 1.0 | 1.97 | 0.048 | 5.6 |
| **Age implant.** | 0.99 | 0.01 | -0.66 | 0.50 | 1.0 |
| **Sexe** | 2.28 | 1.01 | 1.85 | 0.064 | 5.4 |
| **Site Mitral** | 0.23 | 0.10 | -3.15 | 0.002 | 0.57 |
| **Nombre bio.** | 1.28 | 0.57 | 0.56 | 0.57 | 3.0 |

La prise en compte du groupe ABO permet également de limiter l'incidence des altérations précoces des bioprothèses avant 7 ans. De la même façon les patients du groupe A ont un risque moins élevé de dégénérescence précoce de leur bioprothèse (avant 7 ans) que les patients des autres groupes.

### 4.3 Dans cette invention nous démontrons comment la réactivité des patients vis à vis de résidus sucrés portées par les bioprothèses peut expliquer la propension des bioprothèses à calcifier.

Au moment de l'explantation l'état calcifié ou pas de la bioprothèse a été colligé. Il faut savoir qu'il existe d'autres types de dégénérescence pour les bioprothèses avec notamment les déchirures ou la formation d'un pannus.

Différent facteurs ont été rapportés pour expliquer la tendance à la calcification notamment l'âge du patient à l'implantation avec une calcification particulièrement rapide chez le sujet jeune, la durée d'implantation de la bioprothèse. Nous démontrons là encore l'importance du groupe ABO avec une moindre tendance à la calcification pour les patients du groupe A (coef. <1. cf. Tableau 6)

**Tableau 6**

| **Calification O/N** | **Coef.** | **Std Err.** | **T** | **P(t)** | **Interval. 95%** |
|---|---|---|---|---|---|
| **Durée implant**. | 0.96 | 0.02 | -1.16 | 0.24 | 1.02 |
| **Groupe A** | 0.72 | 0.15 | -1.55 | 0.12 | 1.08 |
| **Age Implant**. | 0.96 | 0.006 | -4.87 | 0.0002 | 0.98 |

Les résultats des analyses multivatiées comparant les autres groupes O par rapport aux autres groupes, B et AB sont rapportés dans le tableau 7. Si l'effet du groupe O sur la calcification est indifférent, les patients du groupe B et AB ont une plus forte tendance à calcifier leur bioprothèse (cf. coef.>1 1.3 et 1.12 (supérieurs à 1) respectivement).

**Tableau 7 :**

| **Calcification O/N** | **Coef.** | **Std Err.** | **T** | **P(t)** | **Interval. 95%** |
|---|---|---|---|---|---|
| **Groupe O** | 1.0 | 0.04 | 0.18 | 0.96 | 1.4 |
| **Groupe B** | 1.3 | 0.36 | 1.10 | 0.79 | 2.2 |
| **Groupe AB** | 1.12 | 0.46 | 0.28 | 0.78 | 2.5 |

### Exemple 5 : Le phénotype ABH du tissu bioprothétique doit être adapté au phénotype ABO/ABH du patient:

En plus de l'exemple d'implantation chez l'animal (exemple 3), où nous avons montré que la calcification du tissu bloprothétique chez l'animal dépend de son phénotype ABH et celui de la bioprothèse implantée, nous avons recherché si cela se vérifiait également chez l'homme. Nous avons vérifié si les résultats exceptionnels n'étaient pas en fait liés à une adéquation par le fait du hasard entre le phénotype ABH de la bioprothèse et le phénotype ABO du patient.

Nous avons recherché sur les bioprothèses porcines à longévité exceptionnelle (supérieure à 16 ans) le phénotype ABH du porc à partir duquel les bioprothèses avaient été fabriquées. Nous avons donc extrait l'ADN des bioprothèses explantées en utilisant le Kit de Invitrogen pour extraire l'ADN. Nous avons alors recherché l'expression de l'activité A transferase L'ADN a été amplifié en utilisant 3 paires de « primers » décrite pour amplifier le gènes A1 humain (56). Si le gène codant pour l'enzyme A transferase porcine et l'humaine est identique, les fragments de restrictions sont différents et il est donc possible en réalisant une digestion des produits de PCR par *EcoRI* avec ou sans *BamHI (56)* et une migration sur gel d'agarose d'authentifier l'origine de l'activité A transférase. Ainsi pour un patient de type A il est possible de détecter une activité A transferase d'origine porcine. D'autres marqueurs exprimés chez le porc et chez l'homme mais de taille différente comme le gène P53 ont été utilisés comme contrôle.

L'analyse des différentes bioprothèses explantées à longévité exceptionnelle a montré que les patients du groupe A qui ont une bioprothèse à longévité exceptionnelle ont tous reçu une bioprothèse provenant de porc de type A. Pour les quelques patients des groupes B et O qui ont eu une bioprothèse à longévité exceptionnelle, aucun n'a reçu de bioprothèse de type A.

Cette analyse démontre donc qu'il est fondamental d'avoir une adéquation entre le phénotype ABH du tissu animal et le phénotype ABO du patient.

Cette invention propose donc des bioprothèses adaptées au phénotype ABO des patients.

## Revendications

1. Procédé d'obtention d'une bioprothèse médicale fixée chimiquement implantable chez un patient humain, comprenant des substances qui ont été prélevées de tissu animal non-humain, le procédé comprenant une étape au cours de laquelle on sélectionne positivement une bioprothèse fixée chimiquement pour son implantation dans le corps dudit patient humain lorsque (i) le type phénotypique dans le système ABO/ABH de ladite bioprothèse fixée chimiquement est compatible avec (ii) le type phénotypique dans le système ABO/ABH dudit patient humain, les bioprothèses medicales étant choisies parmi les valves cardiaques, les bioprothèses cardiaques, les bioprothèses artérielles, les bioprothèses vasculaires, un patch ou tissu, les bioprothèses pulmonaires, les tissus de remplacement ou de régénération ou association.

2. Procédé selon la revendication 1, comprenant les étapes suivantes:
a) fournir une pluralité de bioprothèses fixées chimiquement dont le type phénotypique dans le système ABO/ABH est connu, et,
b) sélectionner positivement, au sein de la pluralité desdites bioprothèses fixées chimiquement, au moins une bioprothèse fixée chimiquement pour son implantation dans le corps dudit patient humain lorsque (i) le type phénotypique dans le système ABO/ABH de ladite bioprothèse fixée chimiquement est compatible avec (ii) le type phénotypique dans le système ABO/ABH dudit patient humain.

3. Procédé selon l'une des revendications 1 et 2, **caractérisé en ce que** les substances provenant de tissu animal non-humain contenues dans une bioprothèse fixée chimiquement sont choisies parmi des substances provenant de mammifère.

4. Procédé selon la revendication 3, **caractérisé en ce que** lesdites substances sont choisies parmi des substances provenant d'un mammifère choisi parmi un porc, un bovin, un ovin, un kangourou, un phoque, un chameau et un équidé.

5. Procédé selon l'une des revendications 1,4 **caractérisé en ce que** lesdites bioprothèses fixées chimiquement consistent en des valves cardiaques choisies parmi les valves mitrale, aortique, pulmonaire et tricuspide.

6. Procédé selon la revendication 2, **caractérisé en ce que** la sélection d'une bioprothèse est réalisée selon les règles de compatibilité suivantes :
- si le phénotype ABO/ABH dudit patient humain est le phénotype A, on sélectionne une bioprothèse fixée chimiquement dont le type phénotypique ABO/ABH est un phénotype choisi parmi A ou H,
- si le phénotype ABO/ABH dudit patient humain est le phénotype O, on sélectionne une bioprothèse fixée chimiquement dont le type phénotypique ABO/ABH est un phénotype H,
- si le phénotype ABO/ABH dudit patient humain est le phénotype B, on sélectionne une bioprothèse fixée chimiquement dont le type phénotypique ABO/ABH est un phénotype choisi parmi B (humain) ou H, et
- si le phénotype ABO/ABH dudit patient humain est le phénotype AB, on sélectionne une bioprothèse fixée chimiquement dont le type phénotypique ABO/ABH est un phénotype choisi parmi A, B ou H.

7. Procédé selon l'une des revendications 1 à 7, **caractérisé en outre en ce que**,
- le phénotype dans le système Lewis, respectivement (i) pour la ou les bioprothèse(s) candidate(s) fixée(s) chimiquement et (ii) pour le patient humain , est déterminé ou connu, et
- la sélection comprend la selection d'une bioprothèse fixée chimiquement si plus d'une comptabilité est établie pour le type phénotypique dans le système Lewis.

8. Procédé selon la revendication 1, **caractérisé en ce que** ladite bioprothèse est fixée chimiquement au moyen d' un agent réticulant.

9. Procédé selon la revendication 9, **caractérisé en ce que** ledit agent réticulant cqmporte du glutaraldehyde.

## Patentansprüche

1. Verfahren zur Herstellung einer chemisch fixierten medizinischen Bioprothese, die in einen menschlichen Patienten implantierbar ist, umfassend Substanzen, die tierischem, nicht menschlichem Gewebe entnommen wurden, wobei das Verfahren einen Schritt umfasst, bei dem eine chemisch fixierte Bioprothese für ihre Implantation in den Körper des menschlichen Patienten positiv ausgewählt wird, wenn (i) der Phänotyp im System ABO/ABH der chemisch fixierten Bioprothese mit (ii) dem Phänotyp-Typ im System ABO/ABH des menschlichen Patienten kompatibel ist, wobei die medizinischen Bioprothesen aus den Herzklappen, den Herz-Bioprothesen, den arteriellen Bioprothesen, den vaskulären Bioprothesen, einem Patch oder Gewebe, den Lungen-Bioprothesen, den Ersatz- oder Regenerations- oder Assoziationsgeweben ausgewählt sind.

2. Verfahren nach Anspruch 1, das die folgenden Schritte umfasst:
a) Bereitstellen einer Vielzahl chemisch fixierter Bioprothesen, deren Phänotyp-Typ im System AB0/ABH bekannt ist, und
b) positives Auswählen aus der Vielzahl der chemisch fixierten Bioprothesen mindestens einer chemisch fixierten Bioprothese für ihre Implantation in den Körper des menschlichen Patienten, wenn (i) der Phänotyp im System AB0/ABH der chemisch fixierten Bioprothese mit (ii) dem Phänotyp im System AB0/ABH des menschlichen Patienten kompatibel ist.

3. Verfahren nach einem der Ansprüche 1 und 2, **dadurch gekennzeichnet, dass** die Substanzen aus nicht menschlichem tierischem Gewebe, die in einer chemisch fixierten Bioprothese enthalten sind, aus Säugetiersubstanzen ausgewählt sind.

4. Verfahren nach Anspruch 3, **dadurch gekennzeichnet, dass** die Substanzen aus Substanzen aus einem Säugetier ausgewählt sind, das aus einem Schwein, einem Rind, einem Schaf, einem Känguru, einer Robbe, einem Kamel und einem Pferd ausgewählt ist.

5. Verfahren nach einem der Ansprüche 1 bis 4, **dadurch gekennzeichnet, dass** die chemisch fixierten Bioprothesen aus Herzklappen bestehen, die aus den Mitral-, Aorten-, Pulmunal- und Trikuspidalklappen ausgewählt sind.

6. Verfahren nach Anspruch 2, **dadurch gekennzeichnet, dass** die Auswahl einer Bioprothese gemäß den folgenden Kompatibilitätsregeln durchgeführt wird:
- wenn der Phänotyp AB0/ABH des menschlichen Patienten der Phänotyp A ist, wird eine chemisch fixierte Bioprothese ausgewählt, deren Phänotyp-Typ AB0/ABH ein aus A oder H ausgewählter Phänotyp ist,
- wenn der Phänotyp AB0/ABH des menschlichen Patienten der Phänotyp 0 ist, wird eine chemisch fixierte Bioprothese ausgewählt, deren Phänotyp-Typ AB0/ABH ein Phänotyp H ist,
- wenn der Phänotyp AB0/ABH des menschlichen Patienten der Phänotyp B ist, wird eine chemisch fixierte Bioprothese ausgewählt, deren Phänotyp-Typ AB0/ABH ein aus B (menschlich) oder H ausgewählter Phänotyp ist, und
- wenn der Phänotyp AB0/ABH des menschlichen Patienten der Phänotyp AB ist, wird eine chemisch fixierte Bioprothese ausgewählt, deren Phänotyp-Typ AB0/ABH ein aus A, B oder H ausgewählter Phänotyp ist.

7. Verfahren nach einem der Ansprüche 1 bis 7, ferner **dadurch gekennzeichnet, dass**
- der Phänotyp im Lewis-System jeweils (i) für die chemisch fixierten Kandidaten-Bioprothese(n) und (ii) für den menschlichen Patienten bestimmt wird oder bekannt ist, und
- die Auswahl die Auswahl einer chemisch fixierten Bioprothese umfasst, wenn für den Phänotyp-Typ im Lewis-System mehr als eine Kompatibilität vorhanden ist.

8. Verfahren nach Anspruch 1, **dadurch gekennzeichnet, dass** die Bioprothese mit Hilfe eines Vernetzungsmittels chemisch fixiert wird.

9. Verfahren nach Anspruch 9, **dadurch gekennzeichnet, dass** das Vernetzungsmittel Glutaraldehyd aufweist.

## Claims

1. A method to obtain a chemically attachable medical bioprosthesis implantable in a human patient, comprising substances which have been taken from non-human animal tissue, the method comprising a step at which a chemically attachable bioprosthesis is positively selected for implanting in the body of said human patient when (i) the phenotype in the ABO/ABH system of said chemically attachable bioprosthesis is compatible with (ii) the phenotype in the ABO/ABH system of said human patient, the medical bioprostheses being selected from among heart valves, heart bioprostheses, arterial bioprostheses, vascular bioprostheses, a patch or tissue, lung bioprostheses, replacement or regeneration or association tissues.

2. The method according to claim 1 comprising the following steps:
a) providing a plurality of chemically attachable bioprostheses having a known phenotype in the ABO/ABH system; and
b) positively selecting from the plurality of said chemically attachable bioprostheses at least one chemically attachable bioprosthesis for implantation in the body of said human patient when (i) the phenotype in the ABO/ABH system of said chemically attachable bioprosthesis is compatible with (ii) the phenotype in the ABO/ABH system of said human patient.

3. The method according to one of claims 1 and 2 **characterized in that** the substances taken from non-human animal tissue contained in a chemically attachable bioprosthesis are selected from among substances derived from mammalians.

4. The method according to claim 3 **characterized in that** said substances are selected from among substances taken from a mammalian selected from among: porcine, bovine, ovine, kangaroos, seals, camels and Equidae.

5. The method according to one of claims 1 to 4 **characterized in that** said chemically attachable bioprostheses are heart valves selected from among mitral, aortic, pulmonary and tricuspid valves.

6. The method according to claim 2 **characterized in that** the selection of a bioprosthesis is performed in accordance with the following compatibility rules:
- if the ABO/ABH phenotype of said human patient is phenotype A, a chemically attachable bioprosthesis is selected having an ABO/ABH phenotype that is a phenotype selected from among A or H;
- if the ABO/ABH phenotype of said human patient is phenotype 0, a chemically attachable bioprosthesis is selected having an ABO/ABH phenotype that is phenotype H;
- if the ABO/ABH phenotype of said human patient is phenotype B, a chemically attachable bioprosthesis is selected having an ABO/ABH phenotype that is a phenotype selected from among B (human) or H; and
- if the ABO/ABH phenotype of said human patient is phenotype AB, a chemically attachable bioprosthesis is selected having an ABO/ABH phenotype that is a phenotype selected from among A, B or H.

7. The method according to one of claims 1 to 6 further **characterized in that**:
- the phenotype in the Lewis system, respectively (i) for the candidate chemically attachable bioprosthesis(es) and (ii) for the human patient, is determined or known; and
- the selection comprises the selection of a chemically attachable bioprosthesis if more than one compatibility is determined for the phenotype in the Lewis system.

8. The method according to claim 1 **characterized in that** said bioprosthesis is chemically attachable by means of a cross-linking agent.

9. The method according to claim 8 **characterized in that** said cross-linking agent comprises glutaraldehyde.
